# EUROPEAN PATENT SPECIFICATION

(11) **EP 0 739 640 B1**
(45) Date of publication and mention of the grant of the patent: **24.07.2002**
(21) Application number: 96106005.0
(22) Date of filing: 16.04.1996
(51) Int. Cl.: A61M 5/32, B24B 19/16

(54) **A medical hollow needle and a method of producing thereof**
Eine hohle medizinische Nadel und ein Verfahren zu ihrer Produktion
Une aiguille creuse à usage médical et une méthode pour sa production

(30) Priority: 28.04.1995 JP 12929795; 14.06.1995 JP 17143895; 12.01.1996 JP 2198896
(43) Date of publication of application: 30.10.1996
(73) Proprietor: Saito, Yoshikuni, Nasu-gun, Tochigi-ken (JP)
(72) Inventor: Saito, Yoshikuni, Nasu-gun, Tochigi-ken (JP)
(74) Representative: Bauer, Wulf, Dr.

(56) References cited:
- EP-A- 0 739 639
- DE-A- 2 005 519
- FR-A- 1 225 009
- FR-A- 2 218 969
- US-A- 3 308 822

## Description

### BACKGROUND OF THE INVENTION

This invention relates to a medical hollow needle suitable to use as a medical hollow needle, such as an injection needle, a cannular needle or the like, for hypodermic injection, dialysis, intravenous drip or blood-collecting, that is, the needle having a flow path in which such fluid as liquid injection medicine, blood or the like can flow, and also relates to a method of producing thereof.

In a conventional method, a medical hollow needle, such as a needle for hypodermic injection, or a cannular needle has been produced by grinding a cylindrical member having a thin diameter. That is, the top end portion of the cylindrical member is grinded in an oblique direction with respect to the direction of the central axis of the cylindrical member so as to form an open bevel end face open in an oblique direction with respect to the direction of the central axis of the cylindrical member, in this way the medical hollow needle has been produced.

It is known that the thinner the wall thickness of a top edge portion of the cylindrical member is made, that is, the thinner the sharp edge portion of a medical hollow needle is made, the less blood vessel at the injection part of a patient or the like is hurt, and the less pain the patient feels at the injection part, at the time of injection or the like. In a conventional medical hollow needle, in order to extremely make a sharp edge portion thin, such an idea that when an open bevel end face is grinded and formed, grinding is performed in such a manner that the inclined angle of the open bevel end face to the center axis direction of the cylindrical member is extremely made small may be proposed. However, in order to grind extremely making the inclined angle of the open bevel end face small, the area for forming the open bevel end face in the center axis direction of the medical hollow needle, that is, the area for grinding the cylindrical member becomes to be wider. As the result, the strength of the medical hollow needle is decreased since the cylindrical member becomes to be thin within its broad area. That is, it was difficult to extremely make the sharp edge portion thin without decreasing the strength of the medical hollow needle.

An object of the present invention is to provide a medical hollow needle and a method of producing thereof capable of extremely making a sharp edge portion thin without marring the strength of a medical hollow needle, taking the above-mentioned circumstances into consideration.

### SUMMARY OF THE INVENTION

The invention comprises a medical hollow needle as defined in claim 1.

US-A-3 308 822 and EP-A-739 639 disclose needles with geometrically different grindings. The EP-document has the same priority as the present application.

In the medical hollow needle according to the present invention, a fourth bevel grinding face (also called sub-bevel face) is provided in addition to the grinding faces, such as the second and the third bevel grinding face (also called right/left bevel faces), thereby the sharp edge portion is extremely thin. Therefore, when the medical hollow needle according to the present invention having the thin sharp edge portion, thinning the wall thickness of the top edge (also called distal edge) portion, is used, a patient or the like hardly gets hurt in blood vessel at the injection part at the time of execution of injection (that is, since the sharp edge portion is thin and sharp, injury of blood vessel or the like attendant on a stick action is kept at a minimum.), and he can hardly feel the pain at the injection part. Furthermore, since in the medical hollow needle according to the invention, the sharp edge portion is comprised of at least three grinding faces, that is, the second, the third, the fourth bevel grinding faces, the sharp edge portion is located offsetting on the center axis side of the cylindrical member for the fourth bevel grinding face in comparison with a conventional medical hollow needle. Then, when the medical hollow needle according to the present invention is inserted into a blood vessel, the sharp edge portion is located extremely leaving from the inner wall of the blood vessel. As the result, the sharp edge portion is extremely prevented from sticking into the inner wall of a blood vessel, and the pain owing to the injury of the inner wall of a blood vessel and the hurt of a blood vessel is extremely saved. In particular, the pain felt at the portion where a needle is stuck into was a big difficulty in dialysis in which sticking actions by a needle are frequently performed. However, when the medical hollow needle according to the present invention is used as a needle for dialysis, the pain felt at the portion where the needle is stuck into becomes to be little, thereby the difficulty at the time of dialysis can be widely lightened.

Besides, since the sharp edge portion is extremely thin, thinning the wall thickness in the top edge portion of the cylindrical member by providing the fourth bevel grinding face, even if the inclined angle of the second and the third bevel grinding faces to the center axis direction is not extremely made small, the sharp edge portion can be made thin without broadening the area for forming each bevel grinding face in the center axis direction of the medical hollow needle (for instance, the second length L2 and the like), that is, the area for grinding the cylindrical member. Then, the cylindrical member is not thin over its broad area, thereby the strength of the medical hollow needle is not decreased. That is, the medical hollow needle of the present invention is one having the sharp edge portion which becomes to be extremely thin, which strength is not decreased.

Furthermore, the fourth bevel grinding face is provided at the position opposed to the first bevel grinding face. Of the first bevel grinding face, the grinding boundary of the side opposite to the top edge direction of the cylindrical member connects with the cylindrical outer peripheral face. That is, the present invention provides such a structure that the first bevel grinding face is not adjacent to and does not connect with the fourth bevel grinding face on the side opposite to the top edge direction of the cylindrical member. Therefore, for instance, the first bevel grinding face and the fourth bevel grinding face are adjacent to and connect with each other in the top edge direction of the cylindrical member, thereby such inconvenience as the stepped portion which inadvertently hurts blood tissue or the like at the time of injection is formed between the first bevel grinding face and the fourth bevel grinding face can be extremely solved.

When the medical hollow needle according to the invention is used as a metallic needle for a remaining needle, the following effects are exercised.

That is, a remaining needle is assembled in such a manner that a metallic needle and a tube needle made of synthetic resins, such as fluorine contained resin, of its outside are independently produced in advance, and in the last routine the metallic needle is inserted into the tube needle. However, there is such a problem that in a conventional metallic needle, when the metallic needle is inserted into the tube needle, the sharp edge portion (that is, the cutting edge) of the metallic needle is stuck into the inner wall of the tube needle for the reason the inner diameter of the tube needle is extremely small, then the tube needle damages. However, in the metallic needle of the medical hollow needle according to the present invention, the sharp edge portion is comprised of at least three grinding faces, that is, the second, the third, and the fourth bevel grinding faces, thereby the sharp edge portion is located offsetting on the center axis side of the cylindrical member for the forming of the fourth bevel grinding face in comparison with a conventional metallic needle. Therefore, when the metallic needle is inserted into the tube needle, the sharp edge portion of the metallic needle is extremely prevented from being stuck into the inner wall of the tube needle since the sharp edge portion is located extremely leaving from the inner wall of the tube needle, then the damage of the tube needle is extremely saved.

Of the present invention, a 2nd embodiment comprises the medical hollow needle, wherein said sharp edge portion is comprised of four grinding faces, that is, said first, said second, said third and said fourth bevel grinding faces.

In addition to the effects according to the first embodiment, the present embodiment can be also applicable to the needle, such as the so-called back-cut needle, preferably.

Of the present invention, a 3rd embodiment comprises the medical hollow needle of the invention, wherein said fourth bevel grinding face is a part of a tapered grinding face with said center axis as its center.

With this embodiment, at the portion formed the fourth bevel grinding face, near the sharp edge portion, in particular in the peripheral direction of the cylindrical member, the cylindrical outer peripheral face and the like of the cylindrical member are not left adjacent to the fourth bevel grinding face and the like. Therefore, in addition to the effects of the first embodiment, since a stepped portion or the like is not formed in such a state that the fourth bevel grinding face adjoins the cylindrical outer peripheral face in the peripheral direction, the pain at the time of injection can be further saved, conveniently.

Of the present invention, a 4th embodiment comprises the medical hollow needle of the first embodiment, wherein said fourth bevel grinding face is in the shape of a plane. That is, the member in the shape of a cylinder is grinded on a plane, then in the part formed the fourth bevel grinding face, there are the portion which wall thickness is relatively big and the portion which wall thickness is relatively small. That is, in addition to the effects according to the first embodiment, the portion which wall thickness is relatively big is formed. For this reason, the strength of the medical hollow needle is extremely increased. Besides, the portion which wall thickness is relatively small is located near the top edge portion, thereby it is possible to increase the strength of the medical hollow needle and further make the sharp edge portion thin.

Of the present invention, a 5th embodiment comprises the medical hollow needle of the 4th invention, wherein said fourth bevel grinding face extends from said sharp edge portion side to a side opposite to said top end direction of said cylindrical member. That is, the fourth bevel grinding face is formed along the direction in which a needle is stuck into at the time of injection with the medical hollow needle, thereby sticking operation can be extremely smoothly executed without adding inadvertent shock to the stuck blood tissue in addition to the effects of the 4th invention. Therefore, the pain at the time of injection can be further saved, conveniently.

Of the present invention, a 6th embodiment comprises the medical hollow needle of the 5th embodiment, wherein said fourth bevel grinding face is provided such that the positions corresponding to an apical portion of a cylindrical outer peripheral face side of said cylindrical member of a first intersectional portion formed by crossing said first bevel grinding face and said second bevel grinding face and an apical portion of a cylindrical outer peripheral face side of said cylindrical member of a second intersectional portion formed by crossing said first bevel grinding face and said third bevel grinding face, are crossed over by said fourth bevel grinding face in an opposite direction of said top end direction of said cylindrical member. Then, when injection is executed with the medical hollow needle, the apical end portion of the first intersectional portion or the apical end portion of the second intersectional portion might add inadvertent shock to the blood tissue when it passes through the blood tissue stuck. However, the fourth bevel grinding face is provided such that the portions corresponding to these apical end portions extend in the direction opposite to the top end direction of the cylindrical member, that is, the effective diameter of the cylindrical member itself is small by the fourth bevel grinding face at the position corresponding to these apical portions, thereby the inadvertent shock to the before-mentioned blood tissue is extremely softened. Therefore, in addition to the effects of the 5th embodiment, the pain at the time of injection can be further saved, conveniently.

Of the present invention, a 7th embodiment comprises the medical hollow needle of the 5th embodiment, wherein said fourth bevel grinding face is provided such that a position corresponding to said apical portion of the opposite side to said top end direction of said cylindrical member in said grinding boundary of said first bevel grinding face crossed over by said fourth bevel grinding face in a direction opposite to said top end direction of said cylindrical member. Then, when injection is executed with the medical hollow needle, the apical end portion of the grinding boundary might add inadvertent shock to the blood tissue when it passes through the stuck blood tissue. However, the fourth bevel grinding face is provided such that the portion corresponding to this apical end portion extend in the direction opposite to the top end direction of the cylindrical member, that is, the effective diameter of the cylindrical member itself is small by the fourth bevel grinding face at the position corresponding to this apical portion, thereby the inadvertent shock to the before-mentioned blood tissue is extremely softened. Therefore, in addition to the effects of the 5th embodiment, the pain at the time of injection can be further saved, conveniently.

Of the present invention, a 8th embodiment comprises the medical hollow needle of the 4th or the 5th embodiment, wherein said fourth bevel grinding face is provided such that a cylindrical outer peripheral face of said cylindrical member exists between said fourth bevel grinding face and said second bevel grinding face and between said fourth bevel grinding face and third bevel grinding face in a peripheral direction of said cylindrical member. That is, the cylindrical outer peripheral face of the cylindrical member is left without being grinded between the fourth bevel grinding face and the second and the third bevel grinding faces. That is, in addition to the effects of the 4th or the 5th embodiment, the cylindrical outer peripheral face of the cylindrical member is left near the sharp edge portibn without being grinded, thereby the strength of the medical hollow needle in the portion near the sharp edge portion is extremely increased. Besides, the strength of the portion near the sharp edge portion is not marred, thereby it is possible to further make the sharp edge portion thin by extremely making the inclined angle K4 to the center axis of the fourth bevel grinding face small (then, by making the forming area of the fourth bevel grinding face in the center axis direction more wider).

Of the present invention, a 9th embodiment comprises the medical hollow needle of the 1st embodiment, wherein said sharp edge portion is formed positioning on an inner wall forming said flow path of said cylindrical member.

With this embodiment, the ridgeline portion 16 or the like by the second and the third bevel grinding faces is prevented from forming at the portion of wall thickness of the cylindrical member of the sharp edge portion, then the sharp edge portion m(.6 is extremely sharply formed. Therefore, in addition to the effects of the 1st embodiment, the sharp edge portion is extremely sharply formed, thereby the pain at the time of injection can be further saved, conveniently.

Of the present invention, a 10th embodiment comprises the medical hollow needle of the 1st embodiment, wherein said fourth bevel grinding face is formed at an inclined angle 2 -15 degrees with respect to said center axis direction of said cylindrical member.

With this embodiment, in addition to the effects of the 1st embodiment, the sharp edge portion is formed in a proper state in its sharpness and strength, conveniently.

Of the present invention, a 11th embodiment comprises the medical hollow needle of the 1st embodiment, wherein said second and said third inclined angles are equal to each other.

With this embodiment, in addition to the effects of the 1st embodiment, the form of the sharp edge portion is formed equally in right and left, conveniently.

Of the present embodiment, a 12th embodiment comprises the medical hollow needle of the 1st embodiment, wherein said first and said second rotational angles are equal to each other.

With this embodiment, in addition to the effects of the 1st embodiment, the form of the sharp edge portion is formed equally in right and left, conveniently.

Of the present invention, a 13th embodiment comprises a method of producing a medical hollow needle, said method comprising:
performing a grinding machining on a top edge portion of a cylindrical member formed a flow path capable of passing a fluid therein in its center axis direction so as to form a tapered first grinding face reducing an outer diameter toward a top edge direction of said cylindrical member by thinning a wall thickness of said cylindrical member;
performing a grinding machining on said top edge portion of said cylindrical member formed said first grinding face in an oblique direction having a first inclined angle with respect to said center axis so as to form a first bevel grinding face in which said flow path is open in an oblique direction, and performing a grinding machining on said top edge portion of said cylindrical member by rotating a first rotational angle in a positive direction with respect to said first bevel grinding face with said center axis as its center, having a second inclined angle with respect to said center axis so as to form a second bevel grinding face, and by rotating a second rotational angle in an opposite direction with respect to said first bevel grinding face with said center axis as its center, having a third inclined angle with respect to said center axis so as to form a third bevel grinding face, respectively; and forming a sharp edge portion comprised of at least three grinding faces, that is, said second and said third bevel grinding faces, and a fourth bevel grinding face left without being grinded by said grinding machining of said first, said second and said third bevel grinding faces from said first grinding face.

With this embodiment, the fourth bevel grinding face is formed as well as the second and the third bevel grinding faces and the like, different from a conventional medical hollow needle, and the sharp edge portion is comprised of at least these three grinding faces, thereby the sharp edge portion becomes to be extremely thin.

That is, the sharp edge portion becomes to be extremely thin by the fourth bevel grinding face left without being grinded of the first grinding face by forming the first grinding face even if grinding machining is performed so as not to make the inclined angle of the second and the third bevel grinding faces to the center axis direction small. Therefore, the sharp edge portion can be made thin without broadening the area for forming the first, the second, the third, and the fourth bevel grinding faces in the center axis direction of the medical hollow needle, that is, the area for grinding the cylindrical member. Then, the cylindrical member is not made thin for its broader area, and the strength of the medical hollow needle is not decreased. That is, in the method of producing of the present embodiment, the sharp edge portion can be extremely made thin without decreasing the strength of the medical hollow needle.

Besides, since the fourth bevel grinding face is a part of the tapered first grinding face with the center axis as its center, the non-grinding face (the outer peripheral face of the cylindrical member which is not the first, the second, the third or the fourth bevel grinding face, and the like) is not left adjacent to the fourth bevel grinding face and the like at the portion formed the fourth bevel grinding face, near the sharp edge portion, in particular in the peripheral direction of the cylindrical member. Then, a stepped portion or the like is not formed in such a state that the fourth bevel grinding face adjoins the non-grinding face, thereby the pain at the time of injection can be further saved, conveniently.

When grinding is performed on the first, the second, the third bevel grinding faces and the like, the top end portion (also called distal end portion) which is a grinded portion, receives a high heat if the quantity of grinding of one time increases, thereby the material of the top end portion is often softened. When the material of the top end portion is softened, such an inconvenience that the top end portion is chipped off, then the resistance at the time of needle insertion increases and the pain increases, occurs at the time of bur removing work after grinding. However, in the method of producing according to the present invention, the top end portion is grinded so as to form the first grinding face, thereafter the grinding on the first, the second and the third bevel grinding faces is performed. Then, when the first, the second and the third bevel grinding faces are grinded, the quantity of grinding is reduced since the first grinding face is already grinded. Therefore, the top end portion is prevented from receiving a high heat and softening for the reduced quantity of grinding. Then, such an inconvenience that the top end portion is chipped off at the time of bur removing work after grinding is extremely saved.

Of the present invention, a 14th embodiment comprises a method of producing a medical hollow needle, said method comprising:
grinding a top edge portion of a cylindrical member formed a flow path capable of passing a fluid therein in its center axis direction, in an oblique direction with respect to said center axis direction of said cylindrical member, thinning wall thickness of said cylindrical member, so as to form a fourth bevel grinding face in the shape of a plane;
performing a grinding machining on a portion opposed to said fourth bevel grinding face of said top edge portion of said cylindrical member formed said fourth bevel grinding face, in an oblique direction, having a first inclined angle with respect to said center axis so as to form a first bevel grinding face in which said flow path is open in an oblique direction, and performing a grinding machining on said top edge portion of said cylindrical member by rotating a first rotational angle in a positive direction with respect to said first bevel grinding face said center axis as its center, having a second inclined angle with respect to said center axis so as to form a second bevel grinding face, and by rotating a second rotational angle in an opposite direction with respect to said first bevel grinding face with said center axis as its center, having a third inclined angle with respect to said center axis so as to form a third bevel grinding face, respectively; and
forming a sharp edge portion comprised of at least said second, said third and said fourth bevel grinding faces.

With this embodiment, the sharp edge portion becomes to be extremely thin since the fourth bevel grinding face is formed as well as the second and the third bevel grinding faces and the like, different from a conventional medical hollow needle, and the sharp edge portion is comprised of at least these three grinding faces.

That is, the sharp edge portion becomes to be extremely thin by the fourth bevel grinding face by forming the fourth bevel grinding face even if grinding machining is performed so as not to make the inclined angle of the second and the third bevel grinding faces to the center axis direction of the cylindrical member small. Therefore, the sharp edge portion can be made thin without broadening the grinding area of the medical hollow needle in the center axis direction, that is, the area for grinding the cylindrical member. Then, since the cylindrical member is not made thin for its broader area, the strength of the medical hollow needle is not decreased. That is, in the method of producing of the present invention, the sharp edge portion can be extremely made thin without decreasing the strength of the medical hollow needle.

Besides, since in the method of producing in the present embodiment, the first, the second, the third and the fourth bevel grinding faces are grinded in an oblique direction with respect to the center axis direction of the cylindrical member, for instance, it is not necessary to perform a cylindrical grinding or the like. Therefore, in the method of producing of the present embodiment, a grinding machining can be performed with only a grinding machine, such as the grinding machine for forming bevel face grinding (known) which is used when a conventional medical hollow needle is produced, in which grinding is performed by rotating a grinding stone in the shape of a disc, without using the other machines, such as a cylindrical grinder, conveniently.

Furthermore, since the fourth bevel grinding face is formed in the shape of a plane, the non-grinding face (the outer peripheral face of the cylindrical member which is not the first, the second, the third or the fourth bevel grinding face, and the like) is left adjacent to the fourth bevel grinding face in the portion formed the fourth bevel grinding face, near the sharp edge portion, in particular in the peripheral direction of the cylindrical member. Therefore, the strength near the sharp edge portion of the medical hollow needle is extremely increased. Besides, since the non-grinding face is left near the sharp edge portion and the strength near the sharp edge portion is not decreased, the inclined angle K4 of the fourth bevel grinding face to the center axis can be extremely made small (that is, the area formed the fourth bevel grinding face in the center axis direction can be made wider) so as to further make the sharp edge portion thin.

Of the present invention, a 15th embodiment comprises a method of producing a medical hollow needle, said method comprising:
performing a grinding machining on a top edge portion of a cylindrical member formed a flow path capable of passing a fluid therein in its center axis direction, in an oblique direction having a first inclined angle with respect to said center axis so as to form a first bevel grinding face in which said flow path is open in an oblique direction;
performing a grinding machining on said top edge portion of said cylindrical member by rotating a first rotational angle in a positive direction with respect to said first bevel grinding face with said center axis as its center, having a second inclined angle with respect to said center axis so as to form a second bevel grinding face, and by rotating a second rotational angle in an opposite direction with respect to said first bevel grinding face with said center axis as its center, having a third inclined angle with respect to said center axis so as to form a third bevel grinding face, respectively; and
grinding a position opposed to said first bevel grinding face of said top edge portion of said cylindrical member in an oblique direction with respect to said center axis, thinning wall thickness of said cylindrical member so as to form a fourth bevel grinding face in the shape of a plane; and
forming a sharp edge portion comprised of at least said second, said third and said fourth bevel grinding faces.

With this embodiment, in addition to the effects of the 14th embodiment, the work steps till the first, the second and the third bevel grinding faces are formed are almost similar to the producing steps of a conventional medical hollow needle, thereby a production line of a conventional medical hollow needle is easily applicable, conveniently.

Of the present invention, a 16th embodiment comprises a method of producing a medical hollow needle, said method comprising:
performing a grinding machining on a top edge portion of a cylindrical member formed a flow path capable of passing a fluid therein in its center axis direction, in an oblique direction having a first inclined angle with respect to said center axis so as to form a first bevel grinding face in which said flow path is open in an oblique direction;
grinding a position opposed to said first bevel grinding face of said top edge portion of said cylindrical member in an oblique direction with respect to said center axis direction, thinning wall thickness of said cylindrical member so as to form a fourth bevel grinding face in the shape of a plane;
performing a grinding machining on said top edge portion of said cylindrical member by rotating a first rotational angle in a positive direction with respect to said first bevel grinding face with said center axis as its center, having a second inclined angle with respect to said center axis so as to form a second bevel grinding face, and by rotating a second rotational angle in an opposite direction with respect to said first bevel grinding face with said center axis as its center, having a third inclined angle with respect to said center axis so as to form a third bevel grinding face, respectively; and
forming a sharp edge portion comprised of at least said second, said third and said fourth bevel grinding faces.

With this embodiment, in addition to the effects of the 14th embodiment, the work steps till the first bevel grinding face is formed are almost similar to the producing steps of a conventional medical hollow needle, thereby a production line of a conventional medical hollow needle is easily applicable, conveniently.

Of the present invention, a 17th embodiment comprises a method of producing a medical hollow needle, said method comprising:
performing a grinding machining on a top edge portion of a cylindrical member formed a flow path capable of passing a fluid therein in its center axis direction by rotating a first rotational angle in a positive direction with respect to a predetermined standard position with said center axis as its center, having a second inclined angle with respect to said center axis so as to form a second bevel grinding face, and by rotating a second rotational angle in an opposite direction with respect to said standard position with said center axis as its center, having a third inclined angle with respect to said center axis so as to form a third bevel grinding face, respectively;
grinding a position opposed to said standard position of said top edge portion of said cylindrical member in an oblique direction with respect to said center axis direction, thinning wall thickness of said cylindrical member so as to form a fourth bevel grinding face in the shape of a plane;
performing a grinding machining on said top edge portion of said cylindrical member at said standard position in an oblique direction, having a first inclined angle with respect to said center axis so as to form a first bevel grinding face in which said flow path is open in an oblique direction; and
forming a sharp edge portion comprised of at least said second, said third, and said fourth bevel grinding faces.

With this embodiment, in addition to the effects of the 14th embodiment, the work steps till the second and the third bevel grinding faces are formed are almost similar to the producing steps of a conventional medical hollow needle, thereby a production line of a conventional medical hollow needle is easily applicable, conveniently.

Of the present invention, a 18th embodiment comprises a method of producing a medical hollow needle, said method comprising:
performing a grinding machining on a top edge portion of a cylindrical member formed a flow path capable of passing a fluid therein in its center axis direction by rotating a first rotational angle in a positive direction with respect to a predetermined standard position with said center axis as its center, having a second inclined angle with respect to said center axis so as to form a second bevel grinding face, and by rotating a second rotational angle in an opposite direction with respect to said standard position with said center axis as its center, having a third inclined angle with respect to said center axis so as to form a third bevel grinding face, respectively;
performing a grinding machining on said top edge portion of said cylindrical member at said standard position in an oblique direction, having a first inclined angle with respect to said center axis so as to form a first bevel grinding face in which said flow path is open in an oblique direction;
grinding a position opposed to said first bevel grinding face of said top edge portion of said cylindrical member in an oblique direction with respect to said center axis direction, thinning wall thickness of said cylindrical member so as to form a fourth bevel grinding face in the shape of a plane; and
forming a sharp edge portion comprised of at least said second, said third, and said fourth bevel grinding faces.

With this embodiment, in addition to the effects of the 14th embodiment, the work steps till the first, the second and the third bevel grinding faces are formed are almost similar to the producing steps of a conventional medical hollow needle, thereby a production line of a conventional medical hollow needle is easily applicable, conveniently.

Of the present invention, a 19th embodiment comprises the method of producing the medical hollow needle of the 13th, the 14th, the 15th, the 16th, the 17th or the 18th embodiment wherein said sharp edge portion is formed positioning on an inner wall forming said flow path of said cylindrical member. Then, the ridgeline portion 16 or the like by the second and the third bevel grinding faces is prevented from forming at the portion of wall thickness of the cylindrical member of the sharp edge portion, then the sharp edge portion is extremely sharply formed. Therefore, in addition to the effects of the 13th, the 14th, the 15th, the 16th, the 17th or the 18th embodiment, the sharp edge portion is extremely sharply formed, thereby the pain at the time of injection can be further saved, conveniently.

Of the present invention, a 20th embodiment comprises the method of producing the medical hollow needle of the 13th, the 14th, the 15th, the 16th, the 17th or the 18th embodiment wherein said fourth bevel grinding face is grinded with an inclined angle of 2 - 15 degrees with respect to said center axis direction of said cylindrical member.

With this embodiment, in addition to the effects of the 13th, the 14th, the 15th, the 16th, the 17th or the 18th embodiment, the sharp edge portion is formed in a proper state in its sharpness and strength, conveniently.

Of the present invention, a 21st embodiment comprises the method of producing the medical hollow needle of the 13th, the 14th, the 15th, the 16th, the 17th or the 18th embodiment wherein said second and third inclined angles are equal to each other.

With this invention, in addition to the effects of the 13th, the 14th, the 15th, the 16th, the 17th or the 18th embodiment, the form of the sharp edge portion is formed equally in right and left, conveniently.

Of the present invention, a 22nd embodiment comprises the method of producing the medical hollow needle of the 13th, the 14th, the 15th, the 16th, the 17th or the 18th embodiment wherein said first and said second rotational angles are equal to each other.

With this embodiment, in addition to the effects of the 13th, the 14th, the 15th, the 16th, the 17th or the 18th embodiment, the form of the sharp edge portion is formed equally in right and left, conveniently.

Of the present invention, the 23rd embodiment comprises the medical hollow needle of the 1st embodiment, wherein a fifth bevel grinding face (also called right auxiliary grinding face) is provided at an apical portion of a cylindrical outer peripheral face side of said cylindrical member in a first intersectional portion formed by crossing said first bevel grinding face and said second bevel grinding face, by removing said apical portion, and a sixth bevel grinding face (also called left auxiliary grinding face) is provided at an apical portion of a cylindrical outer peripheral face side of said cylindrical member in a second intersectional portion formed by crossing said first bevel grinding face and said third bevel grinding face, by removing said apical portion. Then, in addition to the effects of the 1st embodiment, since the fifth and the sixth bevel grinding faces are provided by respectively removing the both sides of apical portions in the medical hollow needle according to the 23rd embodiment before-mentioned, the insertion resistance with the medical hollow needle is lower than one in which both sides of apical portions are not removed. That is, when the medical hollow needle according to the 23rd embodiment is stuck into a human body or the like at the time of execution of injection, the resistance to a skin or a blood vessel tissue is small for not providing both sides of the apical portions, thereby the hurt of the skin and the blood vessel tissue is hardly caused, then a patient hardly feels pain at the injection part.

Figures 4, 15 and 18 are helpful for understanding the invention but these figures do not fall under claim 1 since the sub-bevel face 12 is not plane.

### BRIEF DESCRIPTION OF THE DRAWINGS

Fig.1 is a side view showing an example of a medical hollow needle according to the present invention;
Fig.2 is a view seen from upper hand of Fig.1;
Fig.3 is a view seen from lower hand of Fig.1;
Fig.4 is a sectional view of line X1-Y1 of Fig.2;
Fig.5 is a side view showing another example of the medical hollow needle according to the present invention;
Fig.6 is a view seen from lower hand of Fig.5;
Fig.7 is a sectional view of line X2-Y2 of Fig.5;
Fig.8 is a side view showing another example of the medical hollow needle according to the present invention;
Fig.9 is a view seen from upper hand of Fig.8;
Fig.10 is a view seen from lower hand of Fig.8;
Fig.11 is a side view showing another example of the medical hollow needle according to the present invention;
Fig.12 is a view seen from lower hand of Fig.11;
Fig.13 is a side view showing another example of the medical hollow needle according to the present invention;
Fig.14 is a view seen from upper hand of Fig.13;
Fig.15 is a view seen from lower hand of Fig.13;
Fig.16 is a side view showing another example of the medical hollow needle according to the present invention;
Fig.17 is a view seen from upper hand of Fig.16;
Fig.18 is a sectional view of line X3-Y3 of Fig.17;
Fig.19 is a side view showing another example of the medical hollow needle according to the present invention;
Fig.20 is a view seen from lower hand of Fig. 19;
Fig.21 is a side view showing another example of the medical hollow needle according to the present invention; and
Fig.22 is a view seen from lower hand of Fig.21.

### DESCRIPTION OF THE PREFERRED EMBODIMENT

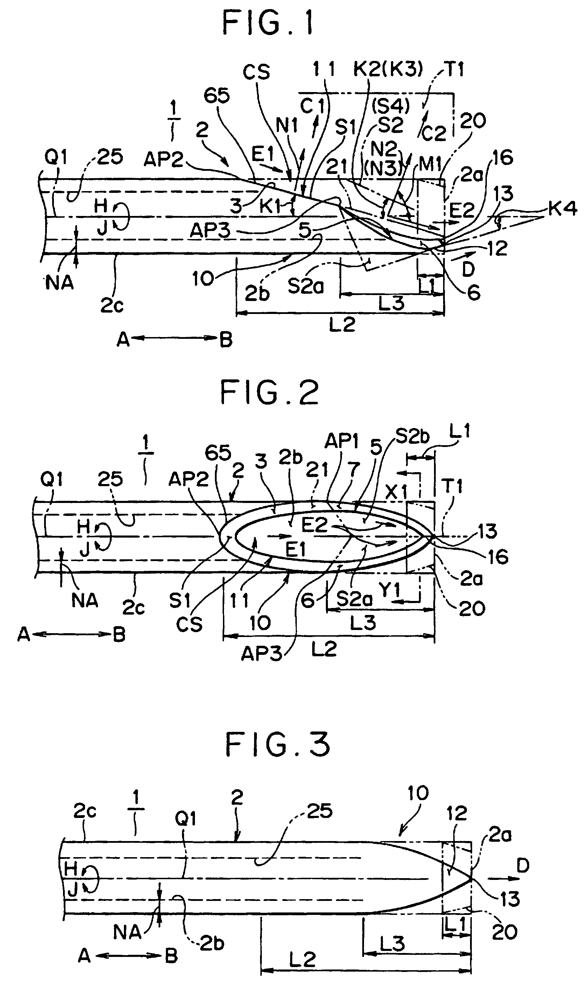

A medical needle 1, or an example of a medical hollow needle according to the present invention, is produced, as shown in Figs.1 through 4, by grinding a cylindrical member 2 (shown by a two-dot chain line in a part) which is a cylindrical bar-shaped member, which diameter is extremely small, extending in the directions as shown by the arrows A and B in respective figures (On this occasion, the direction as shown by the arrow B is a direction of a top end of the cylindrical member 2.). Inside the cylindrical member 2, a medical liquid flow hole 25 in which a fluid, such as a medical liquid for injection, can pass, is formed in the direction of an axis center Q1 of the cylindrical member 2 (or in the directions as shown by the arrows A and B). On the outer peripheral side of the cylindrical member 2, a cylindrical outer peripheral face 2c is basically formed.

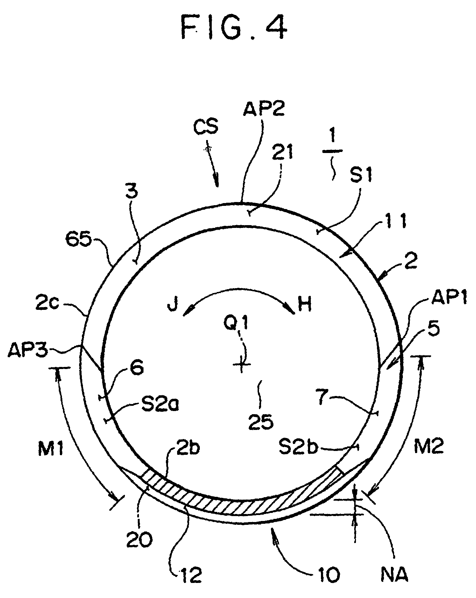

A sub-bevel face 12 is provided with a top end portion 10 of the cylindrical member 2, positioning on the lower side of the paper of Fig.1 and Fig.4. The sub-bevel face 12 comprises a part of a first grinding face 20 formed by taperingly grinding a first length L1 (that is, the area in the directions as shown by the arrows A and B) of a top edge 2a side of the cylindrical member 2 (that is, the edge portion side of the arrow B side of the figure), reducing the outside diameter of the cylindrical member 2 for the direction as shown by the arrow B, coaxially with the cylindrical member 2 (that is, making the shape of a circular truncated cone brought down), then thinning a wall thickness NA of the cylindrical member 2 for the direction as shown by the arrow B. That is, the sub-bevel face 12 is positioned along the direction as shown by the arrow D in the figure which is an oblique direction with respect to the direction as shown by the arrow B, or the direction of the axis center Q1 of the cylindrical member 2 (that is, in the direction of a generatrix of a circular truncated cone by the first grinding face 20).

At the top edge portion 10 of the cylindrical member 2, a first bevel face 3 is formed, the first bevel face 3 is the portion positioned on the left side of the paper of Fig.1 of a second grinding face 21 made by cutting the top end portion 10 of the cylindrical member 2 in such a state that the first grinding face 20 is produced, by a grinding plane S1 where the axis center Q1 of the cylindrical member 2 and a first inclined angle K1 (acute angle) intersect each other, inclining to upper left hand in Fig.1 (In fact, the grinding plane S1 is a curved face close to a plane, but this plane S1 is explained as a plane in the present embodiment as a matter of convenience.), taking the arrow B side off. Therefore, a grinding boundary 65 of the arrow A side of the first bevel face 3 connects with the cylindrical outer peripheral face 2c. The grinding boundary 65 is the linear portion in the shape of C connecting points AP1, AP2 and AP3 with one another, as shown in Fig.2 and Fig.4.

The second grinding face 21 is formed in a second length L2 of the top edge 2a side of the cylindrical member 2 (that is, the area in the directions as shown by the arrows A and B), the second length L2 is broader than the first length L1, and a center position CS showing the position of the first bevel face 3 as a matter of convenience (that is, the position on the upper hand of the axis center Q1 in the paper of Fig.1 and Fig.4) opposes to the sub-bevel face 12. The first bevel face 3 is formed, thereby the medical liquid flow hole 25 is open in an oblique direction in the first bevel face 3.

At the top edge portion 10 of the cylindrical member 2, the first grinding face 20 is formed. Furthermore, a second bevel face 5 formed by cutting the top edge 2a side of the cylindrical member 2 in such a state that the second grinding face 21 is formed by grinding planes S2a and S2b of the figure (these are in fact curved faces akin to a plane, similar to the grinding plane S1, but in the present embodiment each of these is explained as a plane as a matter of convenience.) is formed in a third length L3 of the top edge 2a side of the cylindrical member 2 (that is, the area in the directions as shown by the arrows A and B). The third length L3 is broader than the first length L1, and is narrower than the second length L2.

The grinding plane S2a is a plane rotating a plane S2, where the axis center Q1 of the cylindrical member 2 and a second inclined angle K2 (is an acute angle and is bigger than the first inclined angle K1) intersect each other, capable of having a normal vector N2 existing on the plane T1 (the plane along the paper in Fig.1) the same as a normal vector N1 of the grinding plane S1 (Besides, the plane S2 is a plane inclining to the upper left in Fig. 1), a predetermined first rotational angle M1 in the direction as shown by the arrow J of Fig.1 and Fig.4 with the axis center Q1 as its center. And, the grinding plane S2b is a plane rotating a plane S4, where the axis center Q1 and a third inclined angle K3 (is an acute angle and is bigger than the first inclined angle K1) intersect each other, capable of having a normal vector N3 existing on the plane T1 the same as the normal vector N1 (Besides, the plane S4 is a plane inclining to the upper left in Fig.1), a predetermined second rotational angle M2 in the direction as shown by the arrow H of Fig.4, opposite to the direction as shown by the arrow J with the axis center Q1 as its center. In the embodiment, the second inclined angle K2 is equal to the third inclined angle K3 (then, the plane S2 and the plane S4 are the same plane, and the first rotational angle M1 and the second rotational angle M2 are equal to each other (but, the medical hollow needle according to the present invention is not limiting to only the present embodiment, for example, the second inclined angle K2 and the third inclined angle K3 may be different from each other, or the first rotational angle M1 and the second rotational angle M2 may be different from each other.).

The face made by cutting the top edge 2a side of the cylindrical member 2 in such a state that the first grinding face 20 is made, and besides, the second grinding face 21 is made, by the grinding plane S2a is a right bevel face 6, and the face made by cutting by the grinding plane S2b is a left bevel face 7. That is, the second bevel face 5 is comprised of the right bevel face 6 and the left bevel face 7. In other words, at the top edge portion 10 of the cylindrical member 2, the right bevel face 6 is formed, by rotating it the first rotational angle M1 in a positive direction (that is, the direction as shown by the arrow J) to the first bevel face 3 (that is, to the center position CS) with the axis center Q1 as its center, having the second inclined angle K2 to the axis center, and the left bevel face 7 is formed, by rotating it the second rotational angle M2 in the opposite direction (that is, the direction as shown by the arrow H) to the first bevel face 3 (that is, to the center position CS) with the axis center Q1 as its center, having the third inclined angle K3 to the axis center Q1.

As described heretofore, at the top edge portion 10 of the cylindrical member 2, the first bevel face 3 and the second bevel face 5 are successively formed in the second length L2 in the direction as shown by the arrow B, and an open bevel end face 11 is comprised by the first bevel face 3 and the second bevel face 5. The open bevel end face 11 is also the edge portion of the medical liquid flow hole 25 inside the cylindrical member 2, therefore, the medical liquid flow hole 25 is open outside. This open direction is the direction as shown by the arrow C1 of the figure, which is the direction of the normal vector N1 of the grinding plane S1 forming the first bevel face 3, and the direction as shown by the arrow C2 in the figure, which is the direction of the vector averaging the normal vectors of the grinding planes S2a, S2b forming the second bevel face 5 (that is, the normal vector N2 of the plane S2). Then, the open bevel end face 11 is open in an oblique direction to the direction as shown by the arrow B, which is the extending direction of the top edge of the cylindrical member 2. The open bevel end face 11 is provided on the upper side of the paper of Fig.1, as shown in Fig.1, then the sub-bevel face 12 (the lower side of the paper of Fig.1) is provided on the position opposed to the open bevel end face 11.

Furthermore, the open bevel end face 11 and the sub-bevel face 12 are adjacent to each other on the top edge 2a side of the cylindrical member 2, then, a sharp edge portion 13, which is sharp in the direction as shown by the arrow B, is formed between the open bevel end face 11 and the sub-bevel face 12. That is, the sharp edge portion 13 is formed by the three grinding faces, the right bevel face 6, the left bevel face 7 and the sub-bevel face 12. Then, in the medical needle 1, the sub-bevel face 12 (in a conventional medical hollow needle, the open bevel end face only) is provided as well as the open bevel end face 11, thereby the sharp edge portion 13 is extremely thin.

The medical needle 1 according to the present invention is comprised as described heretofore. The medical needle 1 is produced as follows.

At first, a grinding machining is performed on the first length L1 of the top edge portion 10 of the cylindrical member 2 with an appropriate cylindrical grinder (known) as shown in Figs.1 through 3, coaxially to the cylindrical member 2, reducing the outside diameter of the cylindrical member 2 in the direction as shown by the arrow B, that is, thinning the wall thickness NA of the cylindrical member 2 in the direction as shown by the arrow B, so as to form the tapered first grinding face 20 (shown by a full line in part, and by a two-dot chain line in the other part).

Next, a grinding machining is performed on the top edge portion 10 of the cylindrical member 2, in such a state that the first grinding face 20 is formed, with a proper grinding machine, such as the grinding machine capable of grinding by rotating a grinding stone in the shape of a disc (known) in an oblique direction to the direction as shown by the arrow B in figure (that is, the direction having the first inclined angle K1 to the axis center Q1), that is, in the direction as shown by the arrow E1 of the figure which is the direction along the grinding plane S1 so as to form the second grinding face 21 in the second length L2 (then, the first bevel face 3, which is a part of the second grinding face 21, is made.).

Furthermore, the top edge portion 10 of the cylindrical member 2, in such a state that the first grinding face 20 and the second grinding face 21 are made, is grinded with the grinding machine in an oblique direction to the direction as shown by the arrow B in figure, that is, in the direction as shown by the arrow E2 in figure, which is the direction along the grinding plane S2a or the grinding plane S2b so as to form the right bevel face 6 and the left bevel face 7 in the third length L3, that is, so as to form the second bevel face 5.

The second bevel face 5 is formed by grinding, thereby a part of the second grinding face 21 (the right side of the paper of Fig.1, that is, the portion of the third length L3) is grinded and removed, and the first bevel face 3 which is a part of the second grinding face 21 (the left side of the paper of Fig.1) is formed, remaining without being grinded. That is, the open bevel end face 11 comprised of the first bevel face 3 and the second bevel face 5 is formed.

This open bevel end face 11 (that is, the second grinding face 21 and the second bevel face 5) is grinded and formed, thereby a part of the first grinding face 20 (the portion on the upper side of the paper of Fig.1) is grinded and removed, and the sub-bevel face 12 which is a part of the first grinding face 20 (the lower side of the paper of Fig.1) is formed, remaining without being grinded. And, the sharp edge portion 13 is formed between the open bevel end face 11 and the sub-bevel face 12, then the production of the medical needle 1 finishes.

As described heretofore, in the present embodiment the medical needle 1 can be produced so as to extremely make the sharp edge portion 13 thin, thinning the wall thickness NA of the cylindrical member 2, by the sub-bevel face 12 remaining of the first grinding face 20 without being grinded since the first grinding face 20 is formed, unless grinding machining is performed so as to make the inclined angles K1, K2, K3 of the open bevel end face 11 to the direction as shown by the arrow B small. That is, the medical needle 1 having thin top edge portion can be produced without broadening the second length L2 forming the open bevel end face 11, that is, the area where the cylindrical member 2 is grinded, in the entire length in the direction of the axis center Q1 of the medical needle 1. Therefore, the cylindrical member 2 can be prevented from becoming to be thin in its broad area, then the strength of the medical needle 1 can not be impaired.

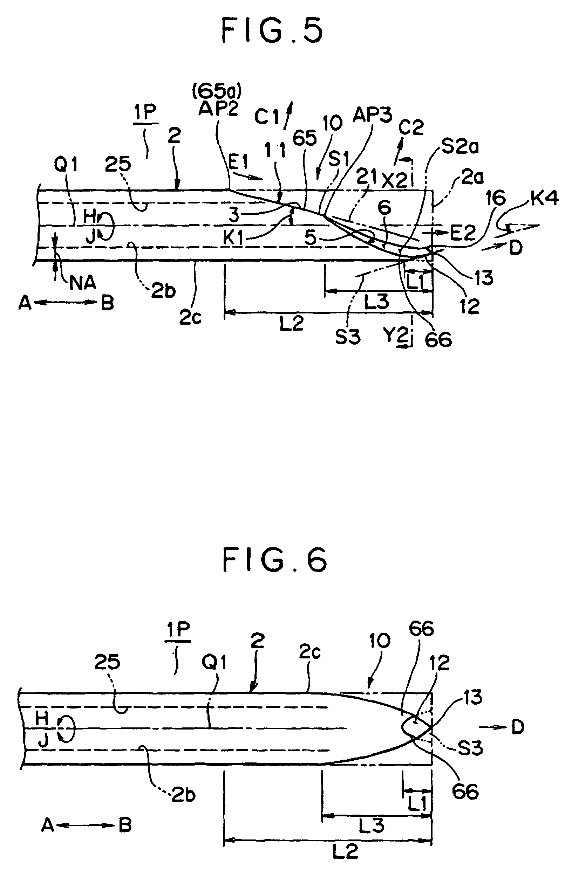

The medical hollow needle according to the present invention may be an another needle different from the medical needle 1 described heretofore. For instance, the medical hollow needle according to the present invention may be a medical needle 1P as shown in Figs.5 through 7.

The portion different from the medical needle 1 described before of the medical needle 1P is the sub-bevel face 12, and this sub-bevel face 12 of the medical needle 1P is in the shape of a plane.

The medical needle 1P is produced as follows.

At first, a grinding machining is performed with a proper grinding machine, such as the grinding machine capable of grinding by rotating a grinding stone in the shape of a disc (known) on the top edge portion 10 of the cylindrical member 2, as shown in Fig.5, in an oblique direction to the direction as shown by the arrow B in the figure which is the direction of the axis center Q1 of the cylindrical member 2, that is, in the direction as shown by the arrow D of Figs. 5 and 6, thinning the wall thickness NA of the cylindrical member 2 in the direction as shown by the arrow B so as to form the sub-bevel face 12 in the shape of a plane made by cutting the top edge portion 10 of the cylindrical member 2 by a grinding plane S3 along the direction as shown by the arrow D (In fact, the sub-bevel face 12 is a curved face close to a plane as a matter of convenience of grinding, but the face 12 can be substantially dealt with a plane.). Between the sub-bevel face 12 and the right bevel face 6, and between the sub-bevel face 12 and the left bevel face 7, that is, in inter-bevel-face areas 66, 66 as shown in Figs.5 through 7, the cylindrical outer peripheral face 2c of the cylindrical member 2 exists in the peripheral direction of the cylindrical member 2.

Thereafter, in a similar way to the case of the medical needle 1 described before, with the grinding machine above-mentioned, grinding machining is performed on the top edge portion 10 of the cylindrical member 2 formed the sub-bevel face 12 in an oblique direction having the first inclined angle K1 with respect to the axis center Q1 in the position opposed to the sub-bevel face 12 (that is, the center position CS of Fig.5), that is, grinding machining is performed in the direction as shown by the arrow E1 of Fig.5, which is the direction along the grinding plane S1 so as to form the second grinding face 21 in the second length L2 (then, the first bevel face 3, where the medical liquid flow hole 25 is open in an oblique direction, is formed as a part of the second grinding face 21.). Furthermore, the top edge portion 10 is grinded in an oblique direction with respect to the direction as shown by the arrow B in the figure, that is, in the direction as shown by the arrow E2 of the figure, which is the direction along the grinding plane S2a or S2b so as to form the right bevel face 6 and the left bevel face 7 in the third length L3, that is, so as to form the second bevel face 5, then the open bevel end face 11 is formed.

This open bevel end face 11 is grinded and formed, thereby the sharp edge portion 13 is formed between the open bevel end face 11 and the sub-bevel face 12, then the production of the medical needle 1P finishes.

The medical needle 1P may be produced by other kinds of steps than one above-mentioned.

For instance, the top edge portion 10 of the cylindrical member 2 is grinded with the grinding machine before-mentioned in an oblique direction having the first inclined angle K1 with respect to the axis center Q1 in the center position CS so as to form the second grinding face 21 in the second length L2 (that is, the first bevel face 3 is formed as a part of the second grinding face 21.). Subsequently, the top edge portion 10 of the cylindrical member 2 is grinded in an oblique direction with respect to the axis center Q1, that is, in the direction as shown by the arrow E2 of the figure, which is the direction along the grinding plane S2a or the grinding plane S2b so as to form the right bevel face 6 and the left bevel face 7 in the third length L3, that is, so as to form the second bevel face 5, then the open bevel end face 11 is formed. Thereafter, the top edge portion 10 is grinded in the position opposed to the first bevel face 3 in an oblique direction with respect to the axis center Q1 in the first length L1 so as to form the sub-bevel face 12 in the shape of a plane made by cutting the top edge portion 10 of the cylindrical member 2 by the grinding plane S3 along the direction as shown by the arrow D. In this way, the sharp edge portion 13 is formed between the open bevel end face 11 and the sub-bevel face 12, then the production of the medical needle 1P finishes.

The medical needle 1P can be also produced by another kind of steps.

For instance, the top edge portion 10 of the cylindrical member 2 is grinded with the grinding machine before-mentioned in an oblique direction having the first inclined angle K1 with respect to the axis center Q1 in the center position CS so as to form the second grinding face 21 in the second length L2 (then, the first bevel face 3 is formed as a part of the second grinding face 21.). Subsequently, the top edge portion 10 is grinded in the first length L1 in an oblique direction with respect to the axis center Q1 in the position opposed to the first bevel face 3 (then, the position opposed to the center position CS) so as to form the sub-bevel face 12 in the shape of a plane made by cutting the top edge portion 10 of the cylindrical member 2 by the grinding plane S3 along the direction as shown by the arrow D. Thereafter, the top edge portion 10 of the cylindrical member 2 is grinded in an oblique direction with respect to the axis center Q1, that is, in the direction as shown by the arrow E2 of the figure, which is the direction along the grinding plane S2a or the grinding plane S2b so as to form the right bevel face 6 and the left bevel face 7 in the third length L3, that is, so as to form the second bevel face 5, then the open bevel end face 11 is formed. In this way, the sharp edge portion 13 is formed between the open bevel end face 11 and the sub-bevel face 12, then the production of the medical needle 1P finishes.

Another kind of step for producing the medical needle 1P will now be explained. For instance, the top edge portion 10 of the cylindrical member 2 is grinded in the direction along the grinding plane S2a or the grinding plane S2b so as to form the right bevel face 6 and the left bevel face 7 in the third length L3, that is, so as to form the second bevel face 5. Thereafter, the top edge portion 10 is grinded in an oblique direction having the first inclined angle K1 with respect to the axis center Q1 in the center position CS so as to form the first bevel face 3. Subsequently, the top edge portion 10 is grinded in the position opposed to the first bevel face 3 (then, the position opposed to the center position CS) in the first length L1 so as to form the sub-bevel face 12 in the shape of a plane made by cutting the top edge portion 10 of the cylindrical member 2 by the grinding plane S3. In this way, the sharp edge portion 13 is formed between the open bevel end face 11 and the sub-bevel face 12, then, the production of the medical needle 1P finishes.

The medical hollow needle according to the present invention may have another form except the medical needles 1, 1P as shown in the respective embodiments above-mentioned.

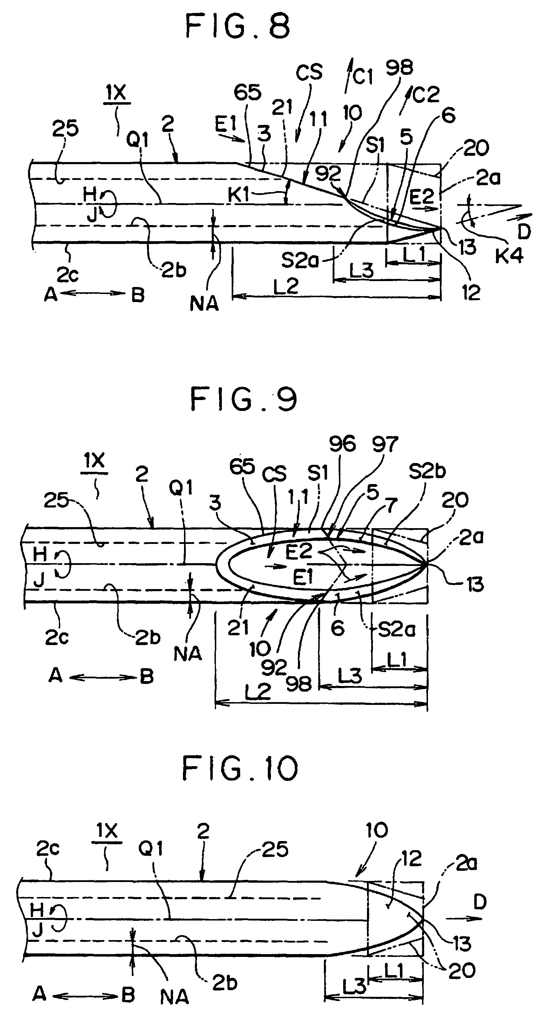

For instance, as shown in Fig.8 through Fig.10, a medical needle 1X has the cylindrical member 2 inside which the medical liquid flow hole 25 is formed in the direction of axis center Q1, basically similar to the medical needle 1 above-mentioned. At the top edge portion 10 of the cylindrical member 2, the first bevel face 3, where the medical liquid flow hole 25 is open in an oblique direction, formed having the first inclined angle K1 with respect to the axis center Q1, is provided on the center position CS. Besides, at the top edge portion 10 of the cylindrical member 2, the right bevel face 6, formed by being rotated at the first rotational angle M1 in the positive direction with respect to the first bevel face 3 with the axis center Q1 as its center, and formed having the second inclined angle K2 with respect to the axis center Q1 is provided, and the left bevel face 7, formed by being rotated at the second rotational angle M2 in the opposite direction to the first bevel face 3 with the axis center Q1 as its center, and formed having the third inclined angle K3 with respect to the axis center Q1, is provided. At the position opposed to the first bevel face 3 of the top edge portion 10, the sub-bevel face 12 grinded and formed in the oblique direction with respect to the axis center Q1 of the cylindrical member 2, thinning the wall thickness NA of the cylindrical member 2, is provided. The sharp edge portion 13 is comprised of the three grinding faces, or the right bevel face 6, the left bevel face 7 and the sub-bevel face 12. Similar to the medical needle 1 above-mentioned, the sub-bevel face 12 is a part of the tapered first grinding face 20 with the axis center Q1 as its center.

However, since in the medical needle 1X, as shown in Figs. 8, 9, the sharp edge portion 13 is formed positioning on an inner wall 2b forming the medical liquid flow hole 25 of the cylindrical member 2, a ridgeline portion 16 (as shown in Figs. 1 and 2) which would be produced by crossing the right bevel face 6 and the left bevel face 7 is not formed near the sharp edge portion 13, then the sharp edge portion 13 is extremely sharply formed.

Since the sub-bevel face 12 in the medical needles 1 and 1X is a part of the tapered first grinding face 20 with the axis center Q1 as its center, in the portion formed the sub-bevel face 12, or the portion near the sharp edge portion 13, especially in the peripheral direction of the cylindrical member 2, as shown in Fig.10 (see Fig.3 concerning the medical needle 1), the cylindrical outer peripheral face 2c of the cylindrical member 2 being adjacent to the sub-bevel face 12, is not left. Therefore, a stepped portion or the like where the sub-bevel face 12 and the cylindrical outer peripheral face 2c would adjoin, is not formed, then the pain at the time of injection can be further prevented conveniently.

Besides, the medical hollow needle according to the present invention may have another form except the medical needles 1, 1P and 1X as shown in the respective embodiment mentioned before.

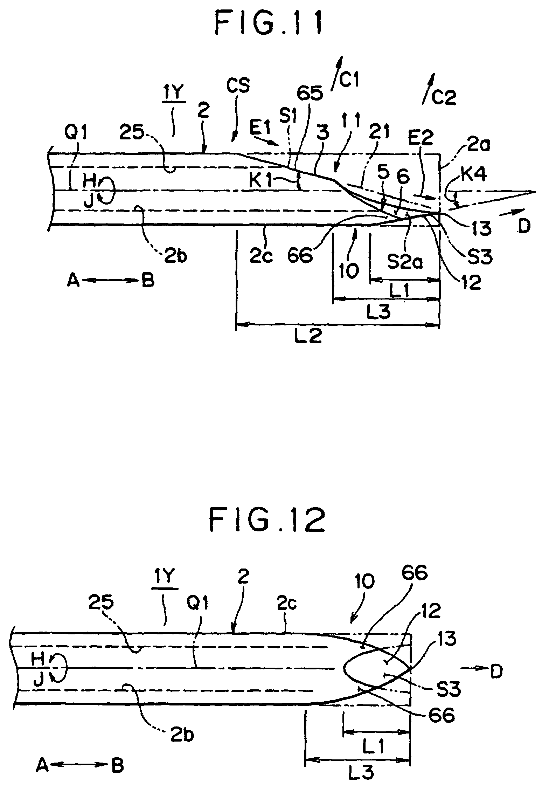

For instance, as shown in Fig.11 and Fig.12, a medical needle 1Y has the cylindrical member 2 inside which the medical liquid flow hole 25 is formed in the direction of the axis center Q1, basically similar to the medical needle 1P before-mentioned. At the top edge portion 10 of the cylindrical member 2, the first bevel face 3, where the medical liquid flow hole 25 is open in an oblique direction, formed having the first inclined angle K1 with respect to the axis center Q1, is provided. Besides, at the top edge portion 10 of the cylindrical member 2, the right bevel face 6, formed by rotating the first rotational angle M1 in the positive direction with respect to the first bevel face 3 with the axis center Q1 as its center, and formed having the second inclined angle K2 with respect to the axis center Q1 is provided, and the left bevel face 7, formed by rotating the second rotational angle M2 in the opposite direction to the first bevel face 3 with the axis center Q1 as its center, and formed having the third inclined angle K3 with respect to the axis center Q1, is provided. At the position opposed to the first bevel face 3 of the top edge portion 10, the sub-bevel face 12 grinded and formed in the oblique direction to the axis center Q1 direction of the cylindrical member 2, thinning the wall thickness NA of the cylindrical member 2, is provided. The sharp edge portion 13 is comprised of the three grinding faces, or the right bevel face 6, the left bevel face 7 and the sub-bevel face 12. The sub-bevel face 12 is in the shape of a plane, similar to the medical needle 1P before-mentioned.

However, since in the medical needle 1Y, as shown in Fig. 11, the sharp edge portion 13 is formed positioning on a inner wall 2b forming the medical liquid flow hole 25 of the cylindrical member 2, a ridgeline portion 16 (as shown in Fig. 5) which would be produced by crossing the right bevel face 6 and the left bevel face 7 is not formed near the sharp edge portion 13, then the sharp edge portion 13 is extremely sharply formed.

The sub-bevel face 12 in the medical needle 1Y is in the shape of a plane in a similar way to the medical needle 1P, and in the portion formed the sub-bevel face 12, or the portion near the sharp edge portion 13, especially in the peripheral direction of the cylindrical member 2, as shown in Figs.11 and 12, the cylindrical outer peripheral face 2c of the cylindrical member 2 is left in the inter-bevel-face areas between the sub-bevel face 12 and the right bevel face 6 and between the sub-bevel face 12 and the left bevel face 7. That is, the cylindrical outer peripheral face 2c is left near the sharp edge portion 13 without being grinded, thereby the strength of the medical needle 1Y in the portion near the sharp edge portion 13 is extremely increased (It's similar way in the before-mentioned medical needle 1P, of course.). Besides, since the cylindrical outer peripheral face 2c is left near the sharp edge portion 13 without being grinded and the strength near the sharp edge portion 13 is not decreased, a sub-bevel inclined angle K4 with respect to the axis center Q1 of the sub-bevel face 12 can be extremely made small (therefore, the area forming the sub-bevel face 12 in the directions as shown by the arrows A and B can be made wider) so as to further make the sharp edge portion 13 thin.

There are results of insertion resistance measuring experiment when a concrete example of the medical needle 1Y before-mentioned is used. That is, insertion resistance measuring experiment has been executed with medical hollow needles, one in which the fourth bevel grinding face, such as the sub-bevel face 12 is formed (that is, the medical needle 1Y) and the other in which the fourth bevel grinding face, such as the sub-bevel face 12, is not formed (that is, the conventional medical hollow needle), and concerning each of these medical hollow needles (the ratio of silicone to flon is 6 percent), the length of face of a cutting edge (that is, the second length L2) is 5.5 mm, the second bevel angle (that is, the second and the third inclined angles K2, K3) is 25.5 - 26 degrees, the length of lancet (that is, the ratio of the third length L3 to the second length L2) is 40 -43 percent, the rotation angle (that is, the first and the second rotational angles M1, M2) is 27.5 degrees, under the condition of room temperature of 25 degrees.

As the results of the experiment, the values of insertion resistance in the needle in which the fourth bevel grinding face is formed, are 150.5g, 156.8g, 136.2g, 149.0g, 137.3g, 151.0g, 137.8g (the average insertion resistance value is 145.5g), and the values of insertion resistance in the needle in which the fourth bevel grinding face is not formed, are 261.2g, 235.6g, 201.3g, 200.7g and 217.1g (the average insertion resistance value is 223.18g). From the results, it is found that the insertion resistance in the medical hollow needle in which the fourth bevel grinding face is formed is smaller than one in the conventional medical hollow needle in which the fourth bevel grinding face is not formed. In the later experiments, it was found that the insertion resistance value is decreased about 40 percent. Therefore, the insertion resistance in the medical hollow needle according to the present invention is small, thereby at the time of execution of injection or the like, a patient or the like gets less hurt in blood vessels and the like at the injection part and feels less pain in the injection part.

In the embodiment before-mentioned, the medical needle of so-called lancet type is shown as the concrete example of the medical hollow needle according to the present invention, but the medical hollow needle according to the present invention may be one except the lancet type. For instance, the medical hollow needle according to the present invention may be the medical needle of so-called K3 type, K3' type, a needle having a lateral hole, and a back-cut type.

A medical needle 1Z of the back-cut type of the medical hollow needles according to the present invention is now explained hereinafter.

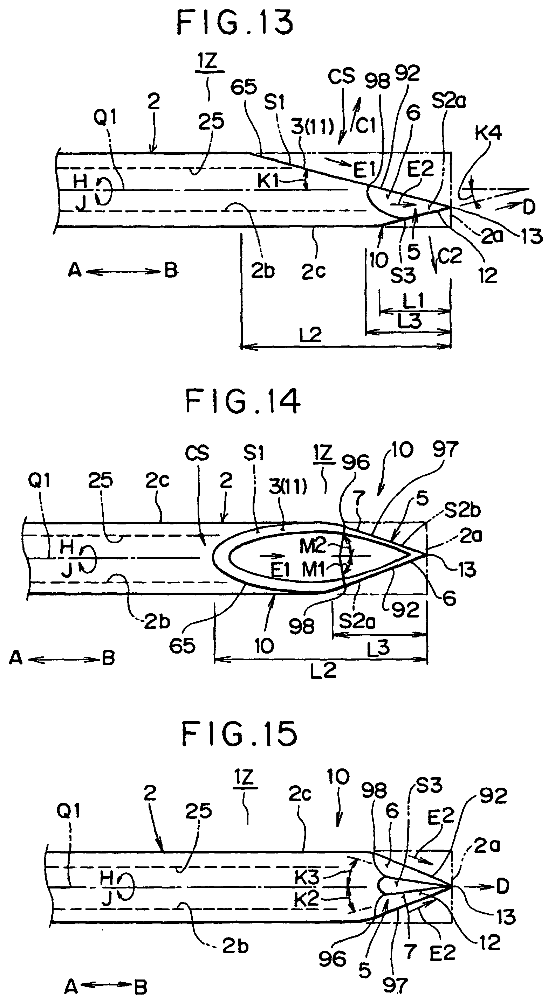

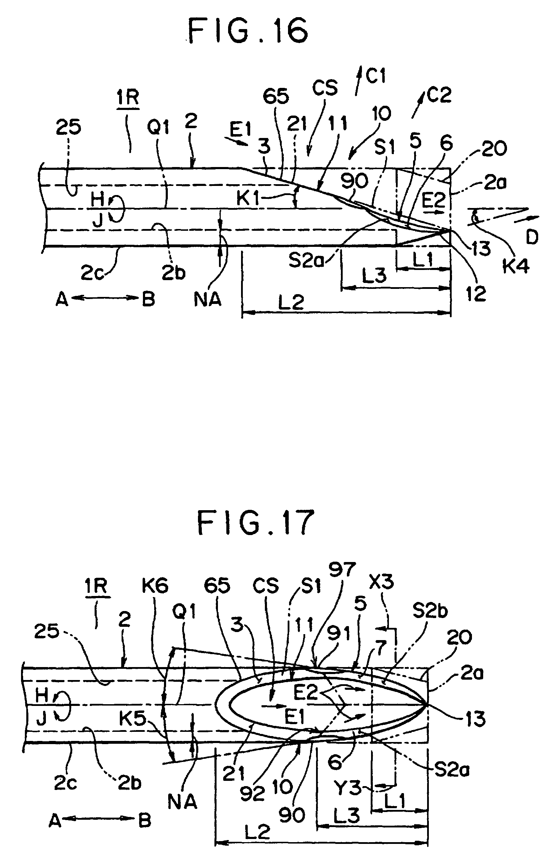

As shown in Figs. 13 through 15, the medical needle 1Z has the cylindrical member 2 inside which the medical liquid flow hole 25 is formed in the direction of axis center Q1. At the top edge portion 10 of the cylindrical member 2, the first bevel face 3, where the medical liquid flow hole 25 is open in an oblique direction, formed having the first inclined angle K1 with respect to the axis center Q1, is provided on the center position CS. On this occasion, the grinding boundary 65 of the arrow A side of the first bevel face 3 connects with the cylindrical outer peripheral face 2c of the cylindrical member 2, in a similar way to the above-mentioned medical needles 1, 1P, 1X and 1Z. Besides, at the top edge portion 10 of the cylindrical member 2, the right bevel face 6, formed by rotating the first rotational angle M1 (90 degrees or more than 90 degrees) in the positive direction with respect to the first bevel face 3 with the axis center Q1 as its center (then, to the center position CS), and formed having the second inclined angle K2 with respect to the axis center Q1 is provided, and the left bevel face 7, formed by rotating the second rotational angle M2 (90 degrees or more than 90 degrees, In the present embodiment, it is shown that the first rotational angle M1 and the second rotational angle M2 are equal with each other, but the first rotational angle M1 and the second rotational angle M2 may be different from each other) in the opposite direction to the first bevel face 3 (then, to the center position CS) with the axis center Q1 as its center, and formed having the third inclined angle K3 with respect to the axis center Q1, is provided (In the present embodiment, the second inclined angle K2 and the third inclined angle K3 are equal to each other, but both angles K2 and K3 may be different from each other). Then, the second bevel face 5 comprised of the right bevel face 6 and the left bevel face 7 is located at the position opposed to the first bevel face 3, and therefore, the open bevel end face 11 by only the first bevel face 3 is formed. At the position opposed to the first bevel face 3 of the top edge portion 10, the sub-bevel face 12 in the shape of a plane, grinded and formed in the oblique direction with respect to the axis center Q1 of the cylindrical member 2, thinning the wall thickness NA of the cylindrical member 2, is provided respectively adjoining to the right bevel face 6 and the left bevel face 7 on both sides, as shown in Fig.15. The sharp edge portion 13 is comprised of the four grinding faces, or the first bevel face 3, the right bevel face 6, the left bevel face 7 and the sub-bevel face 12.

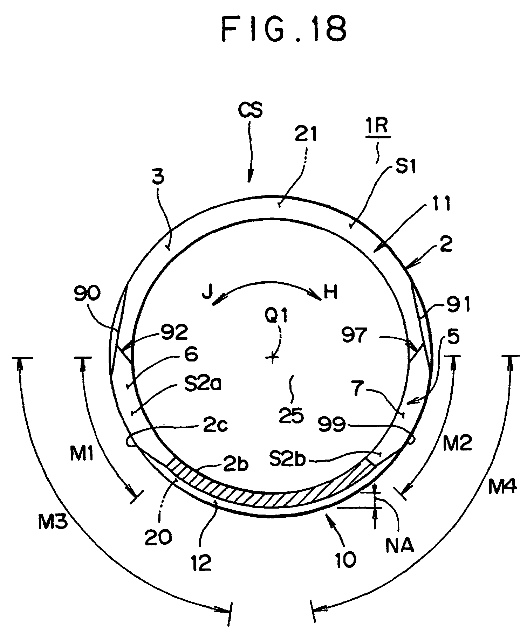

In case where the medical hollow needle according to the present invention is the medical needle of so-called LANCET type, such as the medical needles 1, 1P, 1X and 1Y shown in the before-mentioned embodiments, the medical needle can be comprised of a medical needle 1R, for instance, as shown in Figs. 16 through 18 except the medical needles 1, 1P, 1X and 1Y.

That is, the medical needle 1R of the lancet type has the cylindrical member 2 inside which the medical liquid flow hole 25 is formed in the direction of the axis center Q1, basically similar to the medical needle 1X before-mentioned, as shown in Fig.16 through Fig.18. At the top edge portion 10 of the cylindrical member 2, the first bevel face 3, where the medical liquid flow hole 25 is open in an oblique direction, formed having the first inclined angle K1 with respect to the axis center Q1, is provided on the center position CS. Besides, at the top edge portion 10 of the cylindrical member 2, the right bevel face 6, formed by rotating the first rotational angle M1 in the positive direction with respect to the first bevel face 3 with the axis center Q1 as its center, and formed having the second inclined angle K2 with respect to the axis center Q1 is provided, and the left bevel face 7, formed by rotating the second rotational angle M2 in the opposite direction to the first bevel face 3 with the axis center Q1 as its center, and formed having the third inclined angle K3 with respect to the axis center Q1, is provided. At the position opposed to the first bevel face 3 of the top edge portion 10, the sub-bevel face 12 grinded and formed in the oblique direction with respect to the axis center Q1 of the cylindrical member 2, thinning the wall thickness NA of the cylindrical member 2, is provided. The sharp edge portion 13 is comprised of the three grinding faces, or the right bevel face 6, the left bevel face 7 and the sub-bevel face 12. The sub-bevel face 12 is a part of the tapered first grinding face 20 with the axis center Q1 as its center, similar to the medical needle 1X before-mentioned, the sharp edge portion 13 is formed positioning on the inner wall 2b forming the medical liquid flow hole 25 of the cylindrical member 2.

The medical needle 1R has the following structure in addition to the structure before-mentioned. That is, at the top edge portion 10 of the cylindrical member 2, a right auxiliary grinding face 90 and a left auxiliary grinding face 91 are provided. The right auxiliary grinding face 90 and the left auxiliary grinding face 91 are grinded and formed after the first bevel face 3, the right bevel face 6, the left bevel face 7 and the sub-bevel face 12 are grinded and formed (therefore, after the needle becomes to be similar condition to the medical needle 1X before-mentioned) (This grinding order is not important in the present invention. Any order is applicable.). That is, the right auxiliary grinding face 90 is provided by removing a right apical portion 98 (shown in Fig.8 or Fig.9) of the cylindrical outer peripheral face 2c side of the cylindrical member 2 of a right intersectional portion 92 formed by crossing the first bevel face 3 and the right bevel face 6, and the left auxiliary grinding face 91 is provided by removing a left apical portion 96 (shown in Fig.9) of the cylindrical outer peripheral face 2c side of the cylindrical member 2 of a left intersectional portion 97 formed by crossing the first bevel face 3 and the left bevel face 7. Besides, the right auxiliary grinding face 90 is formed by rotating the third rotational angle M3 in the positive direction with respect to the first bevel face 3 with the axis center Q1 as its center, having a fifth inclined angle K5, which is almost intermediate angle between the first inclined angle K1 and the second inclined angle K2, with respect to the axis center Q1. The left auxiliary grinding face 91 is formed by rotating a fourth rotational angle M4 in the opposite direction to the first bevel face 3 with the axis center Q1 as its center, having a sixth inclined angle K6 which is almost intermediate angle between the first inclined angle K1 and the third inclined angle K3, with respect to the axis center Q1.

Since the right auxiliary grinding face 90 and the left auxiliary grinding face 91 are formed in the medical needle 1R by respectively removing the right apical portion 98 and the left apical portion 96 as described hereinbefore, insertion resistance with the medical needle 1R is lower than the needle, such as the medical needle 1X having the right apical portion 98 and the left apical portion 96. That is, when the medical needle 1R is stuck into a human body or the like at the time of execution of injection or the like, the resistance to a skin or blood tissue is made low by not providing the right apical portion 98 and the left apical portion 96, thereby a patient gets litte hurt at the skin and the blood tissue, then little pain is felt at the injection part.

Furthermore, the medical hollow needle having the fifth bevel grinding face, such as the right auxiliary grinding face 90, and the sixth bevel grinding face, such as the left auxiliary grinding face 91, can be applied to any form of a needle having the apical portions 96 and 98 of the outer peripheral side of the intersectional portions 92, 97 formed by the first bevel face and the second bevel face (that is, the right bevel face 6 and the left bevel face 7), such as the back-cut type in addition to the lancet type before-mentioned. Of course, it is also applicable to the medical needles 1P and 1Y which are ones of a lancet type and which sub-bevel faces 12 are in the shape of a plane, and the medical needles 1 and 1P at each of which the sharp edge portion 13 is not on the inner wall 2b and the ridgeline portion 16 is formed.

For instance, in the medical needle 1Z of the back-cut type as shown in Fig.13 through Fig.15, the right apical portion 98 of the cylindrical outer peripheral face 2c side of the cylindrical member 2 of the right intersectional portion 92 formed by crossing the first bevel face 3 and the right bevel face 6 is grinded and removed so as to provide the fifth bevel grinding face, and the left apical portion 96 of the cylindrical outer peripheral face 2c side of the cylindrical member 2 of the left intersectional portion 97 formed by crossing the first bevel face 3 and the left bevel face 7 is grinded and removed so as to provide the sixth bevel grinding face. The fifth and the sixth bevel grinding faces are provided with the medical hollow needle of the back-cut type, thereby the effects similar to ones of the medical needle 1R before-mentioned are exercised.

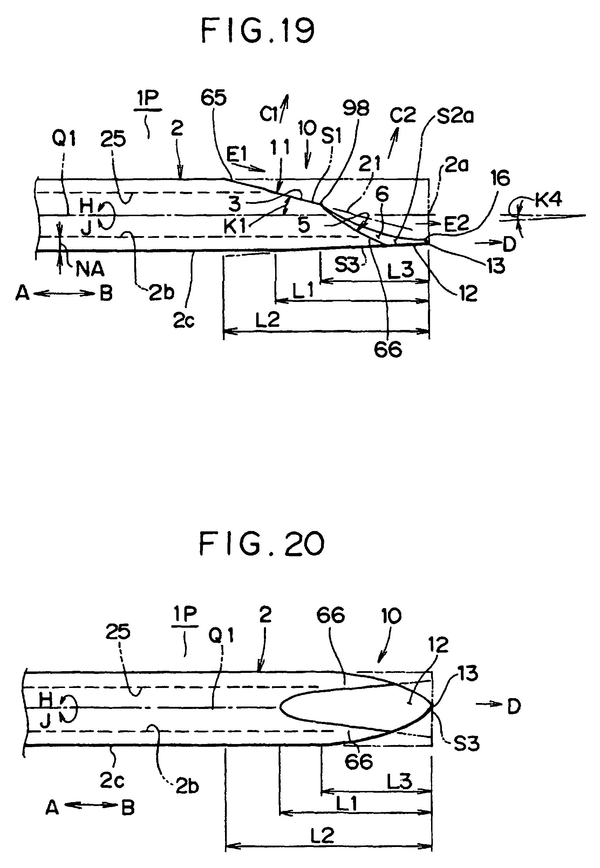

In the medical needle 1P and the like before-mentioned, the sub-bevel face 12 in the shape of a plane is formed. However, the form of the sub-bevel face 12 may have the shape extending from the sharp edge portion 13 side to the arrow A side, as shown in Fig.19 through Fig.22.

In this case, as shown in Figs. 19 and 20, in the sub bevel face 12, the positions corresponding to the right apical portion 98 of the cylindrical outer peripheral face 2c side of the cylindrical member 2 of the right intersectional portion 92 formed by crossing the first bevel face 3 and the right bevel face 6, and the left apical portion 96 of the cylindrical outer peripheral face 2c side of the cylindrical member 2 of a left intersectional portion 97 formed by crossing the first bevel face 3 and the left bevel face 7, may cross over in the opposite direction to the direction of the top end of the cylindrical member 2, that is, in the direction as shown by the arrow A.

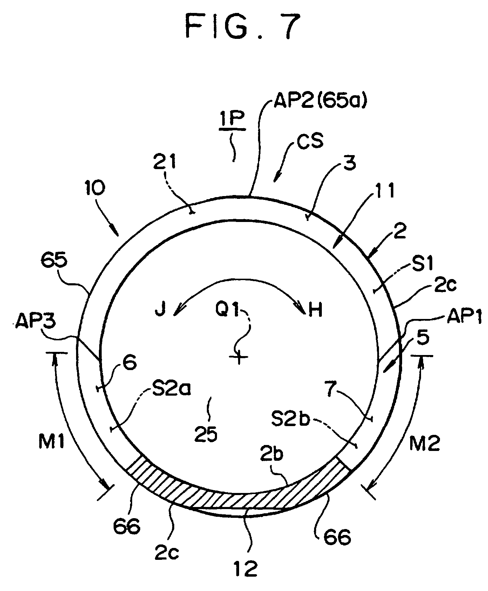

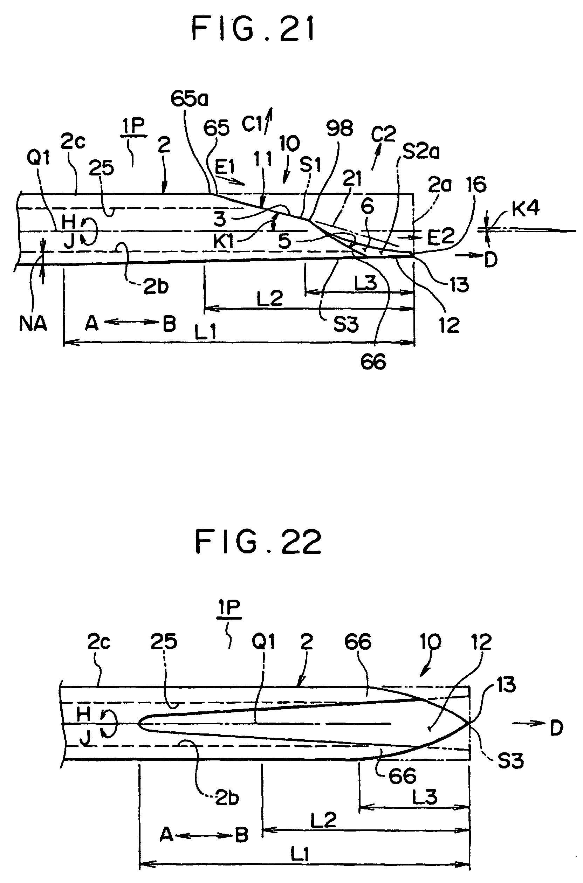

Besides, as shown in Figs. 21 and 22, in the sub bevel face 12, the position corresponding to an apical portion 65a (shown in Fig.5 and Fig.7 also) on the side opposite to the direction of the top end of the cylindrical member 2 of the grinding boundary 65 of the first bevel face 3, that is, on the arrow A side, may cross over in the opposite direction to the direction of the top end of the cylindrical member 2, that is, in the direction as shown by the arrow A.

The medical hollow needle according to the present invention can be applied to various needles, such as a needle for blood-collecting, a dental needle, and a metallic needle of a remaining needle used for blood transfusion or dialysis in addition to the needle for hypodermic injection.

In the medical needles 1, 1P, 1X, 1Y, 1Z and 1R as shown in the respective embodiments before-mentioned, the sub-bevel inclined angle K4 of the sub-bevel face 12 to the direction of the axis center Q1 of the cylindrical member 2 is set 2-15 degrees, thereby the sharp edge portion 13 can be formed having a proper state in sharpness and strength.

The present invention has been explained on the basis of the embodiments presented herein. However, the embodiments which are described in the present specification are illustrative and not limiting. The scope of the invention is designated by the accompanying claims and is not restricted by the descriptions of the specific embodiments.

## Claims

1. A medical hollow needle (1) comprising:
• a cylindrical member (2) having a flow path capable of passing a fluid therein in its center axis Q1 direction;
• a first bevel grinding face (3) formed at a distal end (10) of said cylindrical member (2), such that said flow path is open in an oblique direction, having a first inclined angle K1 with respect to said center axis Q1;
• a right bevel face (6) formed at said distal edge portion (2a) of said cylindrical member (2), whereas the right bevel face (6) is geometrically defined by rotating a first rotational angle M1 in a positive direction of a plane S2 positioned along the upper leg of a second inclined angle K2 whose other leg is the center axis Q1 of the cylindrical member (2) and which second inclined angle K2 is - given that the center axis Q1 defines one leg of K1 and one leg of K2 as well - bigger than the first inclined angle K1;
• a left bevel face (7) formed at said distal edge portion (2a) of said cylindrical member (2), whereas the left bevel face (7) is geometrically defined by rotating a second rotational angle M2 in an opposite direction J of said plane S2;
whereby said needle (1) further comprises:
• a sub-bevel face (12) grinded in the shape of a plane and formed at a position opposed to said first bevel grinding face (3) of said distal edge portion (2a) of said cylindrical (2) member in an oblique direction with respect to said center axis Q1 direction of said cylindrical member (2), thinning the wall thickness of said cylindrical member (2); and
• a sharp edge portion (13) comprising at least three bevel faces, including said right bevel (6), said left bevel (7) and said sub-bevel (12) faces;

2. The medical hollow needle (1) as set forth in claim 1, wherein said sharp edge portion (13) is geometrically comprised of four grinding faces, that is, said first bevel face (3), said right bevel face (6), said left bevel face (7) and said sub-bevel face (12).

3. The medical hollow needle (1) as set forth in claim 1, wherein said sub-bevel face (12) extends from said sharp edge portion (13) side to a side opposite to said distal end direction of said cylindrical member (2).

4. The medical hollow needle as set forth in claim 3, wherein said sub-bevel face (12) is provided such that the positions corresponding to an right apical portion (98) of a cylindrical outer peripheral face side (2c) of said cylindrical member (2) of a right intersectional portion (92) formed by crossing said first bevel grinding face (3) and said right bevel face (6) and an left apical portion (96) of a cylindrical outer peripheral face side (2c) of said cylindrical member (2) of a left intersectional portion (97) formed by crossing said first bevel grinding face (3) and said left bevel face (7), are crossed over by said sub-bevel face (12) in an opposite direction of said distal-end direction of said cylindrical member (2).

5. The medical hollow needle as set forth in claim 4, wherein said sub-bevel face (12) is provided such that a position corresponding to said right apical portion (98) of the opposite side to said distal end direction of said cylindrical member (2) in said grinding boundary of said first bevel grinding face (3) is crossed over by said sub-bevel face (12) in a direction opposite to said distal end direction of said cylindrical member (2).

6. The medical hollow needle (1) as set forth in claim 4 or 5, wherein said sub-bevel face (12) is provided such that a cylindrical outer peripheral face (2c) of said cylindrical member (2) exists between said sub-bevel face (12) and said right bevel face (6) and between said sub-bevel face (12) and left bevel face (7) in a peripheral direction of said cylindrical member (2).

7. The medical hollow needle (1) as set fourth in claim 1, wherein said sharp edge portion (13) is formed on an inner wall forming said flow path of said cylindrical member (2).

8. The medical hollow needle (1) as set forth in claim 1, wherein said sub-bevel-grinding face (12) is formed at an inclined angle K4 of 2-15 degrees with respect to said center axis direction Q1 of said cylindrical member (2).

9. The medical hollow needle (1) as set forth in claim 1, wherein said first rotational angle M1 and said second rotational angle M2 are equal to each other.

10. The medical hollow needle (1) as set forth in claim 1, wherein a right auxiliary grinding (90) face is provided at a right apical portion (98) of a cylindrical outer peripheral face side of said cylindrical member (2) in a right intersectional portion (92) formed by crossing said first bevel grinding face (3) and said right bevel face (6), by removing said right apical portion (98), and a left auxiliary grinding face (91) is provided at a left apical portion (96) of a cylindrical outer peripheral face side of said cylindrical member (2) in a left intersectional portion (97) formed by crossing said first bevel grinding face (3) and said left bevel face (7), by removing said left apical portion (96).

11. A method of producing a medical hollow needle (1) as set forth in claim 1, said method comprising:
a) performing a grinding machining on a distal edge portion (2a) of a cylindrical member (2) having a flow path capable of passing a fluid therein in its center axis direction Q1, in an oblique direction with respect to said center axis direction Q1, in an oblique direction with respect to said center
axis direction Q1 of said cylindrical member (2), thinning the wall thickness of said cylindrical member (2), so as to form a sub-bevel face (12) in the shape of a plane;
b) performing a grinding machining on a portion opposed to said sub-bevel face (12) of said distal edge portion (2a) of said cylindrical member (2) so as to form a first bevel grinding face (3) having a first inclined angle K1 with respect to said center axis Q1 in which said flow path is open in an oblique direction,
c) performing a grinding machining on said distal edge portion (2a) of said cylindrical member (2) by rotating a first rotational angle M1 in a positive direction J with respect to said first bevel grinding face (3) of a plane S2 positioned along the upper leg of a second inclined angle K2 whose other leg is the center axis Q1 of the cylindrical member (2) and which second inclined angle K2 is bigger than the first inclined angle K1 so as to form a right bevel face (6);
d) performing a grinding machining on said distal edge portion (2a) of said cylindrical member (2) by rotating a second rotational angle M2 in an opposite direction H with respect to said first bevel grinding face (3) of said plane S2 so as to form a left bevel face (7);
e) forming a sharp edge portion (13) comprised of at least said right, said left and said sub-bevel faces (6), (7), (12).

12. A method of producing a medical hollow needle (1) as set forth in claim 1, said method comprising:
a) performing a grinding machining on a distal edge portion (2a) of a cylindrical member (2) having a flow path capable of passing a fluid therein in its center axis direction Q1, in an oblique direction with respect to said center axis direction Q1 of said cylindrical member (2) having a first inclined angle K1 with respect to said center axis Q1 so as to form a first bevel grinding face (3) in which said flow path is open in an oblique direction;
b) performing a grinding machining on said distal edge portion (2a) of said cylindrical member (2) by rotating a first rotational angle M1 in a positive direction J with respect to said first bevel grinding face (3) of a plane S2 positioned along the upper leg of a second inclined angle K2 whose other leg is the center axis Q 1 of the cylindrical member (2) and which second inclined angle K2 is bigger than the first inclined angle K1 so as to form a right bevel face (6);
c) performing a grinding machining on said distal edge portion (2a) of said cylindrical member (2) by rotating a second rotational angle M2 in an opposite direction H with respect to said first bevel grinding face (3) of said plane S2 so as to form a left bevel face (7);
d) grinding at a position opposed to said first bevel grinding face (3) of said distal edge portion (2a) of said cylindrical member (2) in an oblique direction with respect to said center axis Q1, thinning the wall thickness of said cylindrical member (2) so as to form a sub-bevel face (12) in the shape of a plane;
e) forming a sharp edge portion comprised of at least said right, said left and said sub-bevel faces (6), (7), (12).

13. A method of producing a medical hollow needle (1) as set forth in claim 1, said method comprising:
a) performing a grinding machining on a distal edge portion (2a) of a cylindrical member (2) having a flow path capable of passing a fluid therein in its center axis direction Q1, in an oblique direction with respect to said center axis direction Q1 of said cylindrical member (2) so as to form a first bevel grinding face (3) having a first inclined angle K1 with respect to said center axis Q1 in which said flow path is open in an oblique direction;
b) grinding at a position opposed to said first bevel grinding face (3) of said distal edge portion (2a) of said cylindrical member (2) in an oblique direction with respect to said center axis Q1, thinning the wall thickness of said cylindrical member (2) so as to form a sub-bevel face (12) in the shape of a plane;
c) performing a grinding machining on said distal edge portion (2a) of said cylindrical member (2) by rotating a first rotational angle M 1 in a positive direction J with respect to said first bevel grinding face (3) of a plane S2 positioned along the upper leg of a second inclined angle K2 whose other leg is the center axis Q1 of the cylindrical member (2) and which second inclined angle K2 is bigger than the first inclined angle K1 so as to form a right bevel face (6);
d) performing a grinding machining on said distal edge portion (2a) of said cylindrical member (2) by rotating a second rotational angle M2 in an opposite direction H with respect to said first bevel grinding face (3) of said plane S2 so as to form a left bevel face (7);
e) forming a sharp edge portion comprised of at least three bevel faces, that is said right, said left and said sub-bevel faces (6), (7), (12).

14. A method of producing a medical hollow needle (1) as set forth in claim 1, said method comprising:
a) performing a grinding machining on a distal edge portion (2a) of a cylindrical member (2) having a flow path capable of passing a fluid therein in its center axis direction Q1, by rotating a first rotational angle M1 in a positive direction J with respect to the center axis direction Q1, having a second inclined angle K2 whose other leg is the center axis Q1 of the cylindrical member (2) and which second inclined angle K2 is bigger than a first inclined angle K1 with respect to said center axis Q1 so as to form a right bevel face (6);
b) performing a grinding machining on said distal edge portion (2a) of said cylindrical member (2) by rotating a second rotational angle M2 in an opposite direction H with respect to said first bevel grinding face (3) of said plane S2 so as to form a left bevel face (7);
c) grinding a position of said distal edge portion (2a) of said cylindrical member (2) in an oblique direction with respect to said center axis direction Q1, thinning the wall thickness of said cylindrical member (2) so as to form a sub-bevel face (12) in the shape of a plane;
d) performing a grinding machining on said distal edge portion (2a) of said cylindrical member (2) in an oblique direction, having a first inclined angle K1 with respect to said center axis Q1 so as to form a first bevel grinding face (3) in which said flow path is open in an oblique direction; and
e) forming a sharp edge portion comprised of at least said right, said left, and said sub-bevel faces (6), (7), (12).

15. A method of producing a medical hollow needle (1) as set forth in claim 1, said method comprising:
a) performing a grinding machining on a distal edge portion (2a) of a cylindrical member (2) having a flow path capable of passing a fluid therein in its center axis direction Q1 by rotating a first rotational angle M1 in a positive direction J with respect to said first bevel grinding face (3) of a plane S2 positioned along the upper leg of a second inclined angle K2 whose other leg is the center axis Q1 of the cylindrical member (2) and which second inclined angle K2 is bigger than a first inclined angle K1 so as to form a right bevel face (6);
b) performing a grinding machining on said distal edge portion (2a) of said cylindrical member (2) by rotating a second rotational angle M2 in an opposite direction H with respect to said first bevel grinding face (3) of said plane S2 so as to form a left bevel face (7);
c) performing a grinding machining on said distal edge portion (2a) of said cylindrical member (2) in an oblique direction with respect to said center axis direction Q1 of said cylindrical member (2), having a first inclined angle K1 with respect to said center axis Q1 so as to form a first bevel grinding face (3) in which said flow path is open in an oblique direction;
d) grinding a position opposed to said first bevel grinding face (3) of said distal edge portion (2a) of said cylindrical member (2) in an oblique direction with respect to said center axis direction Q1, thinning the wall thickness of said cylindrical member (2) so as to form a sub-bevel face (12) in the shape of a plane; and
e) forming a sharp edge portion comprised of at least said second, said third, and said sub-bevel grinding faces (6), (7), (12).

16. The method of producing the medical hollow needle (1) as set forth in claim 11, 12, 13, 14 or 15 wherein said sharp edge portion is formed positioning on an inner wall forming said flow path of said cylindrical member (2).

17. The method of producing the medical hollow needle (1) as set forth in claim 11, 12, 13, 14, or 15 wherein said sub-bevel face (12) is grinded with an inclined angle K4 of 2 - 15 degrees with respect to said center axis direction Q1 of said cylindrical member (2).

18. The method of producing the medical hollow needle (1) as set forth in claim 11, 12, 13, 14, or 15 wherein said first and said second rotational angles M1, M2 are equal to each other.

## Patentansprüche

1. Medizinische Hohlnadel (1), umfassend:
• ein zylindrisches Bauteil (2) mit einem Fließweg derart, dass in Richtung seiner Mittelachse Q1 eine Flüssigkeit passieren kann;
• eine erste Schrägkantenschleiffläche (3), die derart an einem distalen Ende (10) des genannten zylindrischen Bauteils (2) angeformt ist, dass der genannte Fließweg eine schräge Öffnung aufweist, die mit der genannten Mittelachse Q1 einen ersten geneigten Winkel K1 bildet;
• eine rechte Schrägkantenschleiffläche (6), die an diesem distalen Kantenabschnitt (2a) des zylindrischen Bauteils (2) ausgebildet ist, wobei die rechte Schrägkantenschleiffläche (6) geometrisch durch Rotation eines ersten Rotationswinkels M1 in eine positive Richtung einer Ebene S2, die entlang eines oberen Schenkels eines zweiten geneigten Winkels K2, dessen anderer Schenkel die Mittelachse Q1 des zylindrischen Bauteils (2) bildet, positioniert ist, und wobei dieser zweite geneigte Winkel K2 - dadurch, dass die Mittelachse Q1 sowohl einen Schenkel von K1 als auch einen Schenkel von K2 definiert - größer als der erste geneigte Winkel K1 ist;
• eine linke Schrägkantenschleiffläche (7), die an diesem distalen Kantenabschnitt (2a) des zylindrischen Bauteils (2) ausgebildet ist, wobei die linke Schrägkantenschleiffläche (7) geometrisch durch Rotation eines zweiten Rotationswinkels M2 in eine entgegengesetzte Richtung J der genannten Ebene S2 definiert ist;
**dadurch gekennzeichnet, dass** die genannte Nadel (1) weiterhin aufweist:
• eine Unter-Schrägkantenfläche (12), die zu einer ebenen Fläche geschliffen und an einer Position geformt ist, die gegenüber der genannten ersten Schrägkantenschleiffläche (3) an dem distalen Kantenabschnitt (2a) des genannten zylindrischen Bauteils (2) in einer schiefen Richtung in Bezug auf die Richtung der genannten Mittelachse Q1 des genannten zylindrischen Bauteils (2) liegt, wobei die Wandstärke des zylindrischen Bauteils (2) verringert wird; und
• einen scharfen Kantenabschnitt (13), der mindestens drei Schrägkantenflächen aufweist, umfassend die genannte rechte Schrägkantenschleiffläche (6), die genannte linke Schrägkantenschleiffläche (7) und die genannte Unterschrägkantenfläche (12).

2. Medizinische Hohlnadel (1) gemäß Anspruch 1, **dadurch gekennzeichnet, dass** der genannte scharfe Kantenabschnitt (13) geometrisch aus vier Schleifflächen gebildet ist, nämlich aus der genannten ersten Schrägkantenschleiffläche (3), der genannten rechten Schrägkantenschleiffläche (6), der genannten linken Schrägkantenschleiffläche (7) und der genannten Unter-Schrägkantenfläche (12).

3. Medizinische Hohlnadel (1) gemäß Anspruch 1, **dadurch gekennzeichnet, dass** die genannte Unter-Schrägkantenfläche (12) sich von dem Bereich des scharfen Kantenabschnitts (13) bis hin zu einem dem distalen Endbereich des genannten zylindrischen Bauteils (2) gegenüberliegenden Bereich erstreckt.

4. Medizinische Hohlnadel gemäß Anspruch 3, **dadurch gekennzeichnet, dass** die Unter-Schrägkantenfläche (12) derart ausgebildet ist, dass sie in einer zur Richtung des distalen Endes des genannten zylindrischen Bauteils (2) entgegengesetzten Richtung quer zu den Stellen angeordnet ist, die einem rechten Apikalabschnitt (98) einer äußeren zylindrischen umfänglichen Seite (2c) des zylindrischen Bauteils (2) in einem rechten, durch Kreuzen der genannten ersten Schrägkantenschleiffläche (3) und der genannten rechten Schrägkantenschleiffläche (6) geformten Kreuzungsabschnitt (92), und einem linken Apikalabschnitt (96) einer äußeren zylindrischen umfänglichen Seite (2c) des genannten zylindrischen Bauteils (2) in einem linken, durch Kreuzen der genannten ersten Schrägkantenschleiffläche (3) und der genannten linken Schrägkantenschleiffläche (7) geformten Kreuzungsabschnitt (97), entsprechen.

5. Medizinische Hohlnadel gemäß Anspruch 4, **dadurch gekennzeichnet, dass** die Unter-Schrägkantenfläche (12) derart ausgebildet ist, dass sie in einer der Richtung des distalen Endes des genannten zylindrischen Bauteils (2) entgegengesetzten Richtung quer zu einer Stelle angeordnet ist, die dem genannten rechten Apikalabschnitt (98) der dem distalen Ende des genannten zylindrischen Bauteils (2) gegenüberliegenden Seite an der genannten Schleifgrenze dieser ersten Schrägkantenschleiffläche (3) entspricht.

6. Medizinische Hohlnadel (1) gemäß einem der Ansprüche 4 oder 5, **dadurch gekennzeichnet, dass** die Unter-Schrägkantenfläche (12) derart ausgebildet ist, dass zwischen dieser Unter-Schrägkantenfläche (12) und der genannten rechten Schrägkantenschleiffläche (6) und zwischen dieser Unter-Schrägkantenfläche (12) und der genannten linken Schrägkantenschleiffläche (7) in einer umfänglichen Richtung des genannten zylindrischen Bauteils (2) eine äußere zylindrische umfängliche Seite (2c) des genannten zylindrischen Bauteils (2) vorgesehen ist.

7. Medizinische Hohlnadel (1) gemäß Anspruch 1, **dadurch gekennzeichnet, dass** der scharfe Kantenabschnitt (13) an einer Innenwand geformt ist, die den genannten Fließweg des zylindrischen Bauteils (2) bildet.

8. Medizinische Hohlnadel (1) gemäß Anspruch 1, **dadurch gekennzeichnet, dass** die Unter-Schrägkantenfläche (12) in einem 2 bis 15° betragenden geneigten Winkel K4 in Bezug auf die genannte Richtung der Mittelachse Q1 des genannten zylindrischen Bauteils (2) ausgebildet ist.

9. Medizinische Hohlnadel (1) gemäß Anspruch 1, **dadurch gekennzeichnet, dass** sich der erste Rotationswinkel M 1 und der genannte zweite Rotationswinkel M2 einander entsprechen.

10. Medizinische Hohlnadel (1) gemäß Anspruch 1, **dadurch gekennzeichnet, dass** eine rechte Zusatzschleiffläche (90) an einem rechten Apikal-Abschnitt (98) einer äußeren zylindrischen umfänglichen Seite des genannten zylindrischen Bauteils (2) in einem rechten, durch Kreuzen der genannten ersten Schrägkantenschleiffläche (3) und der genannten rechten Schrägkantenschleiffläche (6) geformten Kreuzungsabschnitt (92) durch Entfernen des genannten rechten Apikal-Abschnitts (98) vorgesehen ist, und dass eine linke Zusatzschleiffläche (91) an einem linken Apikal-Abschnitt (96) eines Abschnitts der äußeren zylindrischen umfänglichen Seite des genannten zylindrischen Bauteils (2) in einem linken, durch Kreuzen der genannten ersten Schrägkantenschleiffläche (3) und der genannten linken Schrägkantenschleiffläche (7) geformten Kreuzungsabschnitt (97) durch Entfernen des genannten linken Apikal-Abschnitts (96) vorgesehen ist.

11. Verfahren zur Herstellung einer medizinischen Hohlnadel (1) gemäß Anspruch 1, **dadurch gekennzeichnet, dass** das Verfahren wie folgt abläuft:
a) Durchführen eines Maschinenschleifens an einem distalen Kantenabschnitt (2a) eines zylindrischen Bauteils (2) mit einem Fließweg derart, dass in Richtung seiner Mittelachse Q1 eine Flüssigkeit passieren kann, in einer schiefen Richtung in Bezug auf die genannte Richtung der Mittelachse Q1 des genannten zylindrischen Bauteils (2), wobei die Wandstärke des genannten zylindrischen Bauteils (2) zur Formung einer Unter-Schrägkantenfläche (12) in der Form einer Ebene verringert wird;
b) Durchführen eines Maschinenschleifens in einem Bereich gegenüber der genannten Unter-Schrägkantenfläche (12) des genannten distalen Kantenabschnitts (2a) des genannten zylindrischen Bauteils (2) zur Formung einer ersten Schrägkantenschleiffläche (3), die mit der genannten Mittelachse Q1 einen ersten geneigten Winkel K1 bildet, und in der der genannte Fließweg in einer schiefen Richtung geöffnet ist,
c) Durchführen eines Maschinenschleifens an dem genannten distalen Kantenabschnitt (2a) des genannten zylindrischen Bauteils (2) durch Rotation eines ersten Rotationswinkels M 1 in eine positive Richtung J in Bezug auf die genannte erste Schrägkantenschleiffläche (3) einer Ebene S2, die entlang des oberen Schenkels eines zweiten geneigten Winkels K2 positioniert ist, dessen anderer Schenkel die Mittelachse Q1 des zylindrischen Bauteils (2) bildet, und wobei der zweite geneigte Winkel K2 größer als der erste geneigte Winkel K1 ist, um eine rechte Schrägkantenschleiffläche (6) zu schaffen;
d) Durchführen eines Maschinenschleifens an dem genannten distalen Kantenabschnitt (2a) des genannten zylindrischen Bauteils (2) durch Rotation eines zweiten Rotationswinkels M2 in eine entgegengesetzte Richtung H in Bezug auf die genannte erste Schrägkantenschleiffläche (3) der genannten Ebene S2, um eine linke Schrägkantenschleiffläche (7) zu schaffen;
e) Formen eines scharfen Kantenabschnitts (13), der mindestens aus der genannten rechten Fläche, der genannten linken Fläche und der genannten Unter-Schrägkantenfläche (6), (7), (12) gebildet ist.

12. Verfahren zur Herstellung einer medizinischen Hohlnadel (1) gemäß Anspruch 1, **dadurch gekennzeichnet, dass** das Verfahren wie folgt abläuft:
a) Durchführen eines Maschinenschleifens an einem distalen Kantenabschnitt (2a) eines zylindrischen Bauteils (2) mit einem Fließweg derart, dass in Richtung seiner Mittelachse Q1 eine Flüssigkeit passieren kann, in einer schiefen Richtung mit Bezug auf die Richtung der Mittelachse Q1 des genannten zylindrischen Bauteils (2), um eine erste Schrägkantenschleiffläche (3) mit einem ersten geneigten Winkel K1 zur genannten Mittelachse Q1 zu formen, in welcher der genannte Fließweg eine schräge Öffnung aufweist;
b) Durchführen eines Maschinenschleifens an dem genannten distalen Kantenabschnitt (2a) des genannten zylindrischen Bauteils (2) durch Rotation eines ersten Rotationswinkels M 1 in eine positive Richtung J in Bezug auf die genannte erste Schrägkantenschleiffläche (3) einer Ebene S2, die entlang des oberen Schenkels eines zweiten geneigten Winkels K2 positioniert ist, dessen anderer Schenkel die Mittelachse Q1 des zylindrischen Bauteils (2) bildet, und wobei der zweite geneigte Winkel K2 größer als der erste geneigte Winkel K1 ist, um eine rechte Schrägkantenschleiffläche (6) zu schaffen;
c) Durchführen eines Maschinenschleifens an dem genannten distalen Kantenabschnitt (2a) des genannten zylindrischen Bauteils (2) durch Rotation eines zweiten Rotationswinkels M2 in eine entgegengesetzte Richtung H in Bezug auf die genannte erste Schrägkantenschleiffläche (3) der genannten Ebene S2, um eine linke Schrägkantenschleiffläche (7) zu schaffen;
d) Schleifen einer Stelle, die der ersten Schrägkantenschleiffläche (3) eines distalen Endbereichs (2a) des genannten zylindrischen Bauteils (2) gegenüberliegt, in einer schiefen Richtung in Bezug auf die Mittelachse Q1, durch Verringerung der Wandstärke des genannten zylindrischen Bauteils (2), um eine ebenförmige Unter-Schrägkantenfläche (12) zu schaffen;
e) Formen eines scharfen Kantenabschnitts. der mindestens aus der genannten rechten Fläche, der genannten linken Fläche und der genannten Unter-Schrägkantenfläche (6), (7), (12) gebildet ist.

13. Verfahren zur Herstellung einer medizinischen Hohlnadel (1) gemäß Anspruch 1, **dadurch gekennzeichnet, dass** das Verfahren wie folgt abläuft:
a) Durchführen eines Maschinenschleifens an einem distalen Kantenabschnitt (2a) eines zylindrischen Bauteils (2) mit einem Fließweg derart, dass in Richtung seiner Mittelachse Q1 eine Flüssigkeit passieren kann, in einer schiefen Richtung mit Bezug auf die Richtung der Mittelachse Q1 des genannten zylindrischen Bauteils (2), um eine erste Schrägkantenschleiffläche (3) mit einem ersten geneigten Winkel K1 zur genannten Mittelachse Q1 zu formen, in welcher der genannte Fließweg eine schräge Öffnung aufweist;
b) Schleifen einer Stelle, die der ersten Schrägkantenschleiffläche (3) des distalen Kantenabschnitts (2a) des genannten zylindrischen Bauteils (2) gegenüberliegt, in einer schiefen Richtung in Bezug auf die Mittelachse Q1, durch Verringerung der Wandstärke des genannten zylindrischen Bauteils (2), um eine ebenförmige Unter-Schrägkantenfläche (12) zu schaffen;
c) Durchführen eines Maschinenschleifens an dem genannten distalen Kantenabschnitt (2a) des genannten zylindrischen Bauteils (2) durch Rotation eines ersten Rotationswinkels M1 in eine positive Richtung J in Bezug auf die genannte erste Schrägkantenschleiffläche (3) einer Ebene S2, die entlang des oberen Schenkels eines zweiten geneigten Winkels K2 positioniert ist, dessen anderer Schenkel die Mittelachse Q1 des zylindrischen Bauteils (2) bildet, und wobei der zweite geneigte Winkel K2 größer als der erste geneigte Winkel K1 ist, um eine rechte Schrägkantenschleiffläche (6) zu schaffen;
d) Durchführen eines Maschinenschleifens an dem genannten distalen Kantenabschnitt (2a) des genannten zylindrischen Bauteils (2) durch Rotation eines zweiten Rotationswinkels M2 in eine entgegengesetzte Richtung H in Bezug auf die genannte erste Schrägkantenschleiffläche (3) der genannten Ebene S2, um eine linke Schrägkantenschleiffläche (7) zu schaffen;
e) Formen eines scharfen Kantenabschnitts. der mindestens aus drei Schleifflächen gebildet ist, nämlich der genannten rechten Fläche, der genannten linken Fläche und der genannten Unter-Schrägkantenfläche (6), (7), (12) gebildet ist.

14. Verfahren zur Herstellung einer medizinischen Hohlnadel (1) gemäß Anspruch 1, **dadurch gekennzeichnet, dass** das Verfahren wie folgt abläuft:
a) Durchführen eines Maschinenschleifens an einem distalen Kantenabschnitt (2a) eines zylindrischen Bauteils (2) mit einem Fließweg derart, dass in Richtung seiner Mittelachse Q1 eine Flüssigkeit passieren kann, durch Rotation eines ersten Rotationswinkels M1 in eine positive Richtung J in Bezug auf die Richtung der Mittelachse Q1, mit einem zweiten geneigten Winkel K2, dessen anderer Schenkel die Mittelachse Q1 des zylindrischen Bauteils (2) bildet, und wobei der zweite geneigte Winkel K2 größer als der erste geneigte Winkel K1 in Bezug auf die Mittelachse Q1 ist, um eine rechte Schrägkantenschleiffläche (6) zu schaffen;
b) Durchführen eines Maschinenschleifens an dem genannten distalen Kantenabschnitt (2a) des genannten zylindrischen Bauteils (2) durch Rotation eines zweiten Rotationswinkels M2 in eine entgegengesetzte Richtung H in Bezug auf die genannte erste Schrägkantenschleiffläche (3) der genannten Ebene S2, um eine linke Schrägkantenschleiffläche (7) zu schaffen;
c) Schleifen einer Stelle des distalen Kantenabschnitts (2a) des genannten zylindrischen Bauteils (2) in einer schiefen Richtung in Bezug auf die Richtung der Mittelachse Q1 durch Verringerung der Wandstärke des genannten zylindrischen Bauteils (2), um somit eine ebenförmige Unter-Schrägkantenfläche (12) zu bilden;
d) Durchführen eines Maschinenschleifens an dem genannten distalen Kantenabschnitt (2a) des genannten zylindrischen Bauteils (2) in einer schiefen Richtung, die, zur Formung einer ersten Schrägkantenschleiffläche (3), in der der genannte Fließweg in einer schiefen Richtung geöffnet ist, mit der genannten Mittelachse Q1 einen ersten geneigten Winkel K1 einschließt,
e) Formen eines scharfen Kantenabschnitts. der mindestens aus der genannten rechten Fläche, der genannten linken Fläche und der genannten Unter-Schrägkantenfläche (6), (7), (12) gebildet ist.

15. Verfahren zur Herstellung einer medizinischen Hohlnadel (1) gemäß Anspruch 1, **dadurch gekennzeichnet, dass** das Verfahren wie folgt abläuft:
a) Durchführen eines Maschinenschleifens an einem distalen Kantenabschnitt (2a) eines zylindrischen Bauteils (2) mit einem Fließweg derart, dass in Richtung seiner Mittelachse Q1 eine Flüssigkeit passieren kann, durch Rotation eines ersten Rotationswinkels M 1 in eine positive Richtung J in Bezug auf die genannte erste Schrägkantenschleiffläche (3) einer Ebene S2, die entlang des oberen Schenkels eines zweiten geneigten Winkels K2 positioniert ist, dessen anderer Schenkel die Mittelachse Q1 des zylindrischen Bauteils (2) bildet, und wobei der zweite geneigte Winkel K2 größer als ein erster geneigter Winkel K1 ist, um eine rechte Schrägkantenschleiffläche (6) zu schaffen;
b) Durchführen eines Maschinenschleifens an dem genannten distalen Kantenabschnitt (2a) des genannten zylindrischen Bauteils (2) durch Rotation eines zweiten Rotationswinkels M2 in eine entgegengesetzte Richtung H in Bezug auf die genannte erste Schrägkantenschleiffläche (3) der genannten Ebene S2, um eine linke Schrägkantenschleiffläche (7) zu schaffen;
c) Durchführen eines Maschinenschleifens an dem genannten distalen Kantenabschnitt (2a) des genannten zylindrischen Bauteils (2) in einer schiefen Richtung in Bezug auf die Richtung der Mittelachse Q 1 des genannten zylindrischen Bauteils (2) mit einem ersten geneigten Winkel K1 zur Mittelachse Q1 zur Formung einer ersten Schrägkantenschleiffläche (3), in der der genannte Fließweg in einer schiefen Richtung geöffnet ist;
d) Schleifen einer Stelle, die der ersten Schrägkantenschleiffläche (3) eines distalen Kantenabschnitts (2a) des genannten zylindrischen Bauteils (2) gegenüberliegt, in einer schiefen Richtung in Bezug auf die Mittelachse Q1, durch Verringerung der Wandstärke des genannten zylindrischen Bauteils (2), um eine ebenförmige Unter-Schrägkantenfläche (12) zu schaffen; und
e) Formen eines scharfen Kantenabschnitts. der mindestens aus der genannten zweiten Fläche, der genannten dritten Fläche und der genannten Unter-Schrägkantenfläche (6), (7), (12) gebildet ist.

16. Verfahren zur Herstellung einer medizinischen Hohlnadel (1) gemäß den Ansprüchen 11, 12, 13, 14 oder 15, **dadurch gekennzeichnet, dass** der genannte scharfe Kantenabschnitt an einer inneren, den genannten Fließweg des genannten zylindrischen Bauteils bildenden Wand geformt ist.

17. Verfahren zur Herstellung einer medizinischen Hohlnadel (1) gemäß den Ansprüchen 11, 12, 13, 14 oder 15, **dadurch gekennzeichnet, dass** die genannte Unter-Schrägkantenfläche (12) in einem geneigten Winkel K4 von 2 - 15 Grad zur genannten Mittelachse Q1 des genannten zylindrischen Bauteils (2) geschliffen ist.

18. Verfahren zur Herstellung einer medizinischen Hohlnadel (1) gemäß den Ansprüchen 11, 12, 13, 14 oder 15, **dadurch gekennzeichnet, dass** der genannte erste und der genannte zweite Rotationswinkel M1, M2 einander entsprechen.

## Revendications

1. Aiguille creuse (1) à usage médical comprenant:
• un élément cylindrique (2) ayant un conduit permettant le passage d'un fluide dans la direction de son axe central Q1;
• une première face affûtée coupée en biseau (3) formée à une extrémité distale (10) dudit élément cylindrique (2) de telle manière que ledit conduit s'ouvre dans une direction oblique, selon un premier angle d'inclinaison K1 par rapport au dit axe central Q1;
• une face en biseau droite (6) formée dans le bord distal (2a) dudit élément cylindrique (2), la face en biseau droite (6) étant géométriquement définie par la rotation d'un premier angle de rotation M1 dans une direction positive dans un plan S2 placé le long du côté supérieur d'un deuxième angle d'inclinaison K2 dont l'autre côté est constitué par l'axe central Q1 de l'élément cylindrique (2), ledit deuxième angle d'inclinaison K2 étant supérieur au premier angle d'inclinaison K1 étant donné que l'axe central Q1 définit un côté de K1 ainsi qu'un côté de K2;
• une face en biseau gauche (7) formée dans le bord distal (2a) dudit élément cylindrique (2), la face en biseau gauche (7) étant géométriquement définie par la rotation d'un second angle de rotation M2 dans une direction opposée J dans ledit plan S2;
**caractérisée en ce que** ladite aiguille (1) comprend par ailleurs:
• une face située en dessous du biseau (12) qui est affûtée pour former une surface plane et est réalisée en face de la première face affûtée coupée en biseau (3) dans le bord distal (2a) dudit élément cylindrique (2) dans une direction oblique par rapport à la direction de l'axe central Q 1 dudit élément cylindrique (2) en amincissant l'épaisseur de la paroi dudit élément cylindrique (2); et
• un tranchant (13) comprenant au moins trois faces biseautées comportant la face en biseau droite (6), la face en biseau gauche (7) et la face située en dessous du biseau (12).

2. L'aiguille creuse (1) à usage médical selon la revendication 1, **caractérisée en ce que**, géométriquement parlant, le tranchant (13) comprend quatre faces affûtées, notamment la première face coupée en biseau (3), la face en biseau droite (6), la face en biseau gauche (7) et la face située en dessous du biseau (12).

3. L'aiguille creuse (1) à usage médical selon la revendication 1, **caractérisée en ce que** ladite face située en dessous du biseau (12) s'étend depuis la région du tranchant (13) jusqu'à la région située en face de l'extrémité distale dudit élément cylindrique (2).

4. L'aiguille creuse à usage médical selon la revendication 3, **caractérisée en ce que** ladite face située en dessous du biseau (12) est conçue de manière à ce que ladite face située en dessous du biseau (12) est placée, dans un sens opposé au sens de l'extrémité distale dudit élément cylindrique (2), en travers des endroits correspondant à une partie droite (98) de la pointe d'une face périphérique cylindrique extérieure (2c) dudit élément cylindrique (2) dans une partie d'intersection droite (92) formée en faisant se croiser ladite première face affûtée coupée en biseau (3) et ladite face en biseau droite (6) et à une partie gauche (96) de la pointe d'une face périphérique cylindrique extérieure (2c) dudit élément cylindrique (2) dans une partie d'intersection (97) gauche formée en faisant se croiser ladite première face affûtée coupée en biseau (3) et ladite face en biseau gauche (7).

5. L'aiguille creuse à usage médical selon la revendication 4, **caractérisée en ce que** ladite face située en dessous du biseau (12) est conçue de manière à ce que ladite face située en dessous du biseau (12) est placée, dans un sens opposé au sens de l'extrémité distale dudit élément cylindrique (2), en travers d'un endroit correspondant à ladite partie droite (98) de la pointe du côté opposé au sens de l'extrémité distale dudit élément cylindrique (2) à la limite de la zone d'affûtage de la première face affûtée coupée en biseau (3).

6. L'aiguille creuse (1) à usage médical selon l'une des revendications 4 ou 5, **caractérisée en ce que** ladite face située en dessous du biseau (12) est conçue de manière à ce qu'une face périphérique cylindrique extérieure (2c) dudit élément cylindrique (2) est prévue sur le pourtour dudit élément cylindrique (2) entre ladite face située en dessous du biseau (12) et ladite face en biseau droite (6) et entre ladite face située en dessous du biseau (12) et la face en biseau gauche (7).

7. L'aiguille creuse (1) à usage médical selon la revendication 1, **caractérisée en ce que** ledit tranchant (13) est réalisé dans une paroi intérieure formant le conduit dudit élément cylindrique (2).

8. L'aiguille creuse (1) à usage médical selon la revendication 1, **caractérisée en ce que** ladite face située en dessous du biseau (12) est formée à un angle d'inclinaison K4 de 2 à 15 degrés par rapport à la direction de l'axe central Q1 dudit élément cylindrique (2).

9. L'aiguille creuse (1) à usage médical selon la revendication 1, **caractérisée en ce que** le premier angle de rotation M1 et le second angle de rotation M2 sont égaux.

10. L'aiguille creuse (1) à usage médical selon la revendication 1, **caractérisée en ce qu'**une face affûtée d'appoint droite (90) est prévue sur une partie droite (98) de la pointe d'une face périphérique cylindrique extérieure dudit élément cylindrique (2) dans une partie d'intersection droite (92) formée en faisant se croiser ladite première face affûtée coupée en biseau (3) et ladite face en biseau droite (6) en enlevant la partie droite (98) de la pointe et qu'une face affûtée d'appoint gauche (91) est prévue sur une partie gauche (96) de la pointe d'une partie de la face périphérique cylindrique extérieure dudit élément cylindrique (2) dans une partie d'intersection (97) gauche formée en faisant se croiser ladite première face affûtée coupée en biseau (3) et ladite face en biseau gauche (7) en enlevant la partie gauche (96) de la pointe.

11. Une méthode pour la production d'une aiguille creuse (1) à usage médical selon la revendication 1 qui consiste à:
a) effectuer un affûtage dans un bord distal (2a) d'un élément cylindrique (2) ayant un conduit permettant le passage d'un fluide dans la direction de son axe central Q1 dans une direction oblique par rapport à la direction de l'axe central Q1 dudit élément cylindrique (2) en amincissant l'épaisseur de la paroi dudit élément cylindrique (2) de manière à réaliser une face située en dessous du biseau (12) ayant la forme d'une surface plane;
b) effectuer un affûtage dans une partie opposée à ladite face située en dessous du biseau (12) du bord distal (2a) de l'élément cylindrique (2) de manière à former, selon un premier angle d'inclinaison K1 par rapport à l'axe central Q1, une première face affûtée coupée en biseau (3) dans laquelle s'ouvre en direction oblique le conduit,
c) effectuer un affûtage dans le bord distal (2a) dudit élément cylindrique (2) par rotation d'un premier angle de rotation M 1 dans une direction positive J par rapport à la première face affûtée coupée en biseau (3) dans un plan S2 positionné le long du côté supérieur d'un deuxième angle d'inclinaison K2 dont l'autre côté est constitué par l'axe central Q1 de l'élément cylindrique (2), ce deuxième angle d'inclinaison K2 étant supérieur au premier angle d'inclinaison K1 de manière à former une face en biseau droite (6);
d) effectuer un affûtage dans le bord distal (2a) dudit élément cylindrique (2) par rotation d'un deuxième angle de rotation M2 dans une direction H opposée à ladite première face affûtée coupée en biseau (3) dans le plan S2 de manière à former une face en biseau gauche (7);
e) former un tranchant (13) comprenant au moins les faces en biseau gauche et droite (6), (7) et la face située en dessous du biseau (12).

12. Une méthode pour la production d'une aiguille creuse (1) à usage médical selon la revendication 1 qui consiste à:
a) effectuer un affûtage dans un bord distal (2a) d'un élément cylindrique (2) ayant un conduit permettant le passage d'un fluide dans la direction de son axe central Q1 dans une direction oblique par rapport à la direction de l'axe central Q1 dudit élément cylindrique (2) selon un premier angle d'inclinaison K1 par rapport à l'axe central Q1 de manière à former une première face affûtée coupée en biseau (3) dans laquelle s'ouvre en direction oblique le conduit;
b) effectuer un affûtage dans ledit bord distal (2a) de l'élément cylindrique (2) par rotation d'un premier angle de rotation M 1 dans une direction positive J par rapport à la première face affûtée coupée en biseau (3) dans un plan S2 placé le long du côté supérieur d'un deuxième angle d'inclinaison K2 dont l'autre côté est l'axe central Q 1 de l'élément cylindrique (2) et dont le deuxième angle d'inclinaison K2 est supérieur au premier angle d'inclinaison K1 de manière à former une face en biseau droite (6);
c) effectuer un affûtage dans ledit bord distal (2a) de l'élément cylindrique (2) par rotation d'un second angle de rotation M2 dans une direction opposée H par rapport à la première face affûtée coupée en biseau (3) dans un plan S2 de manière à former une face en biseau gauche (7);
d) affûter un endroit situé à l'opposé de ladite première face affûtée coupée en biseau (3) dudit bord distal (2a) de l'élément cylindrique (2) dans une direction oblique par rapport à l'axe central Q1 en amincissant l'épaisseur de la paroi dudit élément cylindrique (2) de manière à former une face située en dessous du biseau (12) ayant la forme d'une surface plane ;
e) former un tranchant comprenant au moins les faces affûtées en biseau droite et gauche (6), (7) et une face située en dessous du biseau (12).

13. Une méthode pour la production d'une aiguille creuse (1) à usage médical selon la revendication 1 qui consiste à:
a) effectuer un affûtage dans un bord distal (2a) d'un élément cylindrique (2) ayant un conduit permettant le passage d'un fluide dans la direction de son axe central Q1 dans une direction oblique par rapport à la direction de l'axe central Q1 dudit élément cylindrique (2) de manière à former une première face affûtée coupée en biseau (3) selon un premier angle d'inclinaison K1 par rapport à l'axe central Q1 dans laquelle s'ouvre en direction oblique le conduit;
b) affûter un endroit situé à l'opposé de ladite première face affûtée coupée en biseau (3) dudit élément cylindrique (2) dans une direction oblique par rapport à l'axe central Q1 en amincissant l'épaisseur de la paroi dudit élément cylindrique (2) de manière à former une face située en dessous du biseau (12) ayant la forme d'une surface plane ;
c) effectuer un affûtage dans ledit bord distal (2a) de l'élément cylindrique (2) par rotation d'un premier angle de rotation M 1 dans une direction positive J par rapport à la première face affûtée coupée en biseau (3) dans un plan S2 placé le long du côté supérieur d'un deuxième angle d'inclinaison K2 dont l'autre côté est l'axe central Q1 de l'élément cylindrique (2) et dont le deuxième angle d'inclinaison K2 est supérieur au premier angle d'inclinaison K1 de manière à former une face en biseau droite (6);
d) effectuer un affûtage dans ledit bord distal (2a) de l'élément cylindrique (2) par rotation d'un second angle de rotation M2 dans une direction opposée H par rapport à la première face affûtée coupée en biseau (3) dans un plan S2 de manière à former une face en biseau gauche (7);
e) former un tranchant comprenant au moins trois faces affûtées, plus précisément les faces affûtées en biseau droite et gauche (6), (7) et la face située en dessous du biseau (12).

14. Une méthode pour la production d'une aiguille creuse (1) à usage médical selon la revendication 1 qui consiste à:
a) effectuer un affûtage dans un bord distal (2a) d'un élément cylindrique (2) ayant un conduit permettant le passage d'un fluide dans la direction de son axe central Q1 par rotation d'un premier angle de rotation M1 dans une direction positive J par rapport à la direction de l'axe central Q1, selon un deuxième angle d'inclinaison K2 dont l'autre côté constitue l'axe central Q1 de l'élément cylindrique (2), ledit deuxième angle d'inclinaison K2 étant supérieur à un premier angle d'inclinaison K1 par rapport à l'axe central Q1 de manière à former une face en biseau droite (6);
b) effectuer un affûtage dans le bord distal (2a) dudit élément cylindrique (2) par rotation d'un deuxième angle de rotation M2 dans une direction H opposée à ladite première face affûtée coupée en biseau (3) dans le plan S2 de manière à former une face en biseau gauche (7);
c) affûter un endroit dudit bord distal (2a) dudit élément cylindrique (2) dans une direction oblique par rapport à la direction de l'axe central Q1 en amincissant l'épaisseur de la paroi dudit élément cylindrique (2) de manière à réaliser une face située en dessous du biseau (12) ayant la forme d'une surface plane ;
d) effectuer un affûtage dans ledit bord distal (2a) de l'élément cylindrique (2) dans une direction oblique selon un premier angle d'inclinaison K1 par rapport à l'axe central Q1 de manière à former une première face affûtée coupée en biseau (3) dans laquelle s'ouvre en direction oblique le conduit ; et
e) former un tranchant comprenant au moins les faces affûtées en biseau droite et gauche (6), (7) et la face située en dessous du biseau (12).

15. Une méthode pour la production d'une aiguille creuse (1) à usage médical selon la revendication 1 qui consiste à:
a) effectuer un affûtage dans un bord distal (2a) d'un élément cylindrique (2) ayant un conduit permettant le passage d'un fluide dans la direction de son axe central Q1 par rotation d'un premier angle de rotation M 1 dans une direction positive J par rapport à la première face affûtée coupée en biseau (3) dans un plan S2 positionné le long du côté supérieur d'un deuxième angle d'inclinaison K2 dont l'autre côté est constitué par l'axe central Q1 de l'élément cylindrique (2), ce deuxième angle d'inclinaison K2 étant supérieur au premier angle d'inclinaison K1 de manière à former une face en biseau droite (6);
b) effectuer un affûtage dans le bord distal (2a) dudit élément cylindrique (2) par rotation d'un deuxième angle de rotation M2 dans une direction H opposée à ladite première face affûtée coupée en biseau (3) dans le plan S2 de manière à former une face en biseau gauche (7);
c) effectuer un affûtage dans le bord distal (2a) dudit élément cylindrique (2) dans une direction oblique par rapport à la direction de l'axe central Q1 dudit élément cylindrique (2) selon un premier angle d'inclinaison K1 par rapport à l'axe central Q1 de manière à former une première face affûtée coupée en biseau (3) dans laquelle s'ouvre en direction oblique le conduit ;
d) affûter un endroit situé à l'opposé de ladite première face affûtée coupée en biseau (3) dudit bord distal (2a) de l'élément cylindrique (2) dans une direction oblique par rapport à la direction de l'axe central Q1 en amincissant l'épaisseur de la paroi dudit élément cylindrique (2) de manière à former une face située en dessous du biseau (12) ayant la forme d'une surface plane ; et
e) former un tranchant comprenant au moins les deuxième et troisième faces affûtées en biseau (6), (7) et la face située en dessous du biseau (12).

16. Méthode pour la production de l'aiguille creuse (1) à usage médical selon l'une des revendications 11, 12, 13, 14 ou 15, **caractérisée en ce que** le tranchant est positionné dans une paroi interne formant le conduit de l'élément cylindrique (2).

17. Méthode pour la production de l'aiguille creuse (1) à usage médical selon l'une des revendications 11, 12, 13, 14 ou 15, **caractérisée en ce que** ladite face située en dessous du biseau (12) est affûtée à un angle d'inclinaison K4 de 2 à 15 degrés par rapport à la direction de l'axe central Q1 dudit élément cylindrique (2).

18. Méthode pour la production de l'aiguille creuse (1) à usage médical selon l'une des revendications 11, 12, 13, 14 ou 15, **caractérisée en ce que** les premier et deuxième angles de rotation M1, M2 sont égaux.
